(19) Europäisches Patentamt
European Patent Office
Office européen des brevets

(11) Veröffentlichungsnummer: **0 259 804 B1**

# (12) EUROPÄISCHE PATENTSCHRIFT

(45) Veröffentlichungstag der Patentschrift: **18.11.93**

(21) Anmeldenummer: **87112990.4**

(22) Anmeldetag: **04.09.87**

Die Akte enthält technische Angaben, die nach dem Eingang der Anmeldung eingereicht wurden und die nicht in dieser Patentschrift enthalten sind.

(51) Int. Cl.⁵: **C07D 498/06**, A61K 31/535, C07D 215/58, C07D 401/04, C07D 413/04, //(C07D498/06, 271:00,221:00)

## (54) Pyrido [3,2,1-ij]-1,3,4-benzoxadiazinderivate, Verfahren zu deren Herstellung, entsprechende pharmazeutische Präparate und im Verfahren verwendbare Zwischenprodukte.

(30) Priorität: **12.09.86 EP 86112619**
**08.08.87 EP 87111507**

(43) Veröffentlichungstag der Anmeldung:
**16.03.88 Patentblatt 88/11**

(45) Bekanntmachung des Hinweises auf die Patenterteilung:
**18.11.93 Patentblatt 93/46**

(84) Benannte Vertragsstaaten:
**AT BE CH DE ES FR GB GR IT LI LU NL SE**

(56) Entgegenhaltungen:
**EP-A- 0 047 005**
**EP-A- 0 101 829**
**EP-A- 0 160 284**
**DE-A- 3 426 486**
**US-A- 4 499 091**

(73) Patentinhaber: **F. HOFFMANN-LA ROCHE AG**
**Postfach 3255**
**CH-4002 Basel(CH)**

(72) Erfinder: **Aoki, Masahiro**
**Kawana 637**
**Fujisawa-shi**
**Kanagawa-ken(JP)**
Erfinder: **Kamata, Miyako**
**Matsukazedai 18-26**
**Chigasaki-shi**
**Kanagawa-ken(JP)**
Erfinder: **Ohtsuka, Tatsuo**
**Green Heights 101**
**Tsujidotai-heidai, 2-1-6**
**Fujisawa-shi Kanagawa-ken(JP)**
Erfinder: **Shimma, Nobuo**
**Midorigahama 7-57**
**Chigasaki-shi**
**Kanagawa-ken(JP)**
Erfinder: **Yokose, Kazuteru**
**Nekozane 3-19**
**16, Urayasu-shi**
**Chiba-ken(JP)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

(74) Vertreter: **Lederer, Franz, Dr. et al**
**Lederer, Keller & Riederer,**
**Patentanwälte,**
**Lucile-Grahn-Strasse 22**
**D-81675 München (DE)**

**Beschreibung**

Die vorliegende Erfindung betrifft neue Pyrido[3,2,1-ij]-1,3,4-benzoxa-diazinderivate, Verfahren zu deren Herstellung, entsprechende pharmazeutische Präparate und im Verfahren verwendbare Zwischenprodukte. Insbesondere betrifft die Erfindung neue Pyrido[3,2,1-ij]-1,3,4-benzoxadiazinderivate der allgemeinen Formel

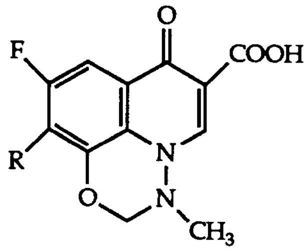

(I)

in der R

- 1-Piperazinyl, das in 4-Stellung durchMethyl, Acetyl oder 4-Aminobenzyl substituiert sein kann;
- Morpholino;
- in 3-Stellung durch Amino, Aminomethyl, (Methyl-amino)methyl, (Aethylamino)methyl, Methoxy oder Chlor substituiertes 1-Pyrrolidinyl;
- 1-Imidazolyl, das in 4-Stellung durch Methyl substituiert sein kann; oder
- in 4-Stellung durch Hydroxy oder Methoxy substituiertes 1-Piperidyl darstellt,

sowie pharmazeutisch verträgliche Salze davon und Hydrate oder Solvate der Verbindungen der Formel I oder von deren Salzen. Diese Produkte sind verwendbar als Wirkstoffe in antibakteriell wirksamen Präparaten.

Die neuen Pyrido[3,2,1-ij]-1,3,4-benzoxadiazinderivate der Formel (I) und deren pharmazeutisch verträgliche Salze sowie die Hydrate oder Solvate davon bzw. von deren Salzen werden erfindungsgemäss durch ein Verfahren hergestellt, welches dadurch gekennzeichnet ist, dass man

(a) eine Verbindung der allgemeinen Formel

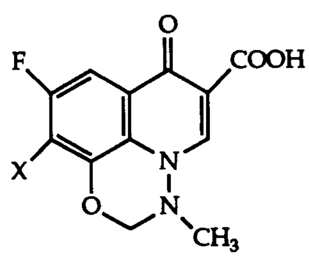

(II)

in der X ein Halogenatom darstellt; und vorhandene Amino-, Hydroxy und/oder Carboxygruppen geschützt sein können,
mit einem Amin der allgemeinen Formel

HR (III)

in der R die oben gegebene Bedeutung hat,
umsetzt und notwendigenfalls eine vorhandene Schutzgruppe abspaltet, oder dass man

(b) eine Verbindung der allgemeinen Formel

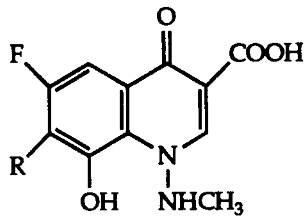

(IV)

in der R die oben gegebene Bedeutung hat; und vorhandene Amino-, Hydroxy-, und/oder Carboxygruppen geschützt sein können,
mit Formaldehyd oder Paraformaldehyd umsetzt und
notwendigenfalls eine vorhandene Schutzgruppe abspaltet, oder dass man
(c) zur Herstellung einer Verbindung der Formel I, worin R 4-Acetyl-1-Piperazinyl darstellt, die Verbindung der Formel I, in der R unsubstituiertes 1-Piperazinylrest darstellt, mit einem die Acetylgruppe abgebenden Mittel umsetzt, oder dass man
(d) zur Herstellung der Verbindung der Formel I, worin R 3-Methoxy-1-piperidyl darstellt, die entsprechende 3-Hydroxy-1-piperidylverbindung der Formel I methyliert, oder dass man
(e) zur Herstellung einer Verbindung der Formel I mit einer freien Aminogruppe R in einer entsprechenden Verbindung der Formel I mit einer geschützten Aminogruppe die Aminoschutzgruppe abspaltet, oder dass man
(f) zur Herstellung der Verbindung der Formel I, worin R 3-Chlor-1-pyrrolidinyldarstellt, die entsprechende 3-Hydroxy-1-pyrrolidinylverbindung mit geschützter Carboxygruppe halogeniert und die Carboxygruppe abspaltet, oder dass man
(g) zur Herstellung der Verbindung der Formel I, worin R 4-(4-Aminobenzyl)-1-piperazinyl darstellt, die Nitrogruppe der entsprechenden 4-(4-Nitrobenzyl)-1-piperazinylverbindung reduziert, oder dass man
(h) zur Herstellung von pharmazeutisch verträglichen Salzen, Hydraten oder Solvaten einer Verbindung der Formel I bzw. von Hydraten und Solvaten besagter Salze, eine Verbindung der Formel I in ein Salz, Hydrat oder Solvat bzw. in ein Hydrat oder Solvat des besagten Salzes überführt.

Verfahren A:

Wie oben angegeben können die erfindungsgemässen Verbindungen erhalten werden durch Umsetzung einer Verbindung der allgemeinen Formel

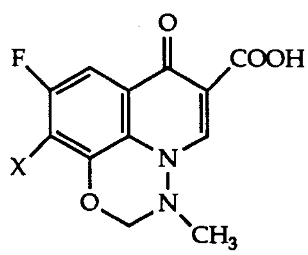

(II)

in der X ein Halogenatom darstellt; und vorhandene Amino-, Hydroxy- und/oder Carboxygruppen geschützt sein können,
mit einem Amin der allgemeinen Formel

HR (III)

in der R die oben gegebene Bedeutung hat,
wonach eine vorhandene Schutzgruppe notwendigenfalls abgespalten wird.

Erfindungsgemäss eingesetzte Verbindungen der Formel (II) sind neue Verbindungen, welche z.B. hergestellt werden können nach den folgenden Reaktionsschema a) oder b).

a)

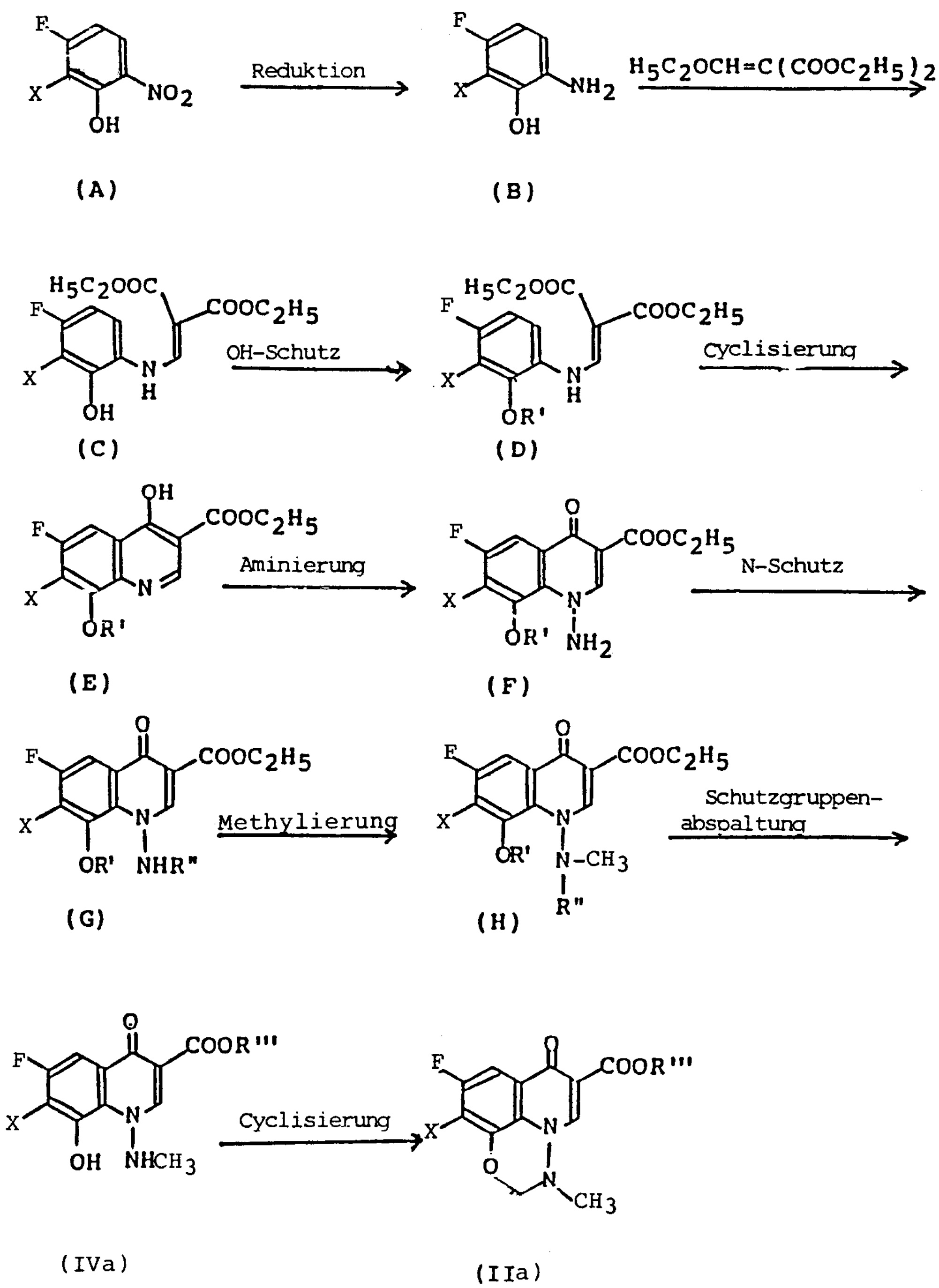


(A) (B) (C) (D) (E) (F) (G) (H) (IVa) (IIa)

b)

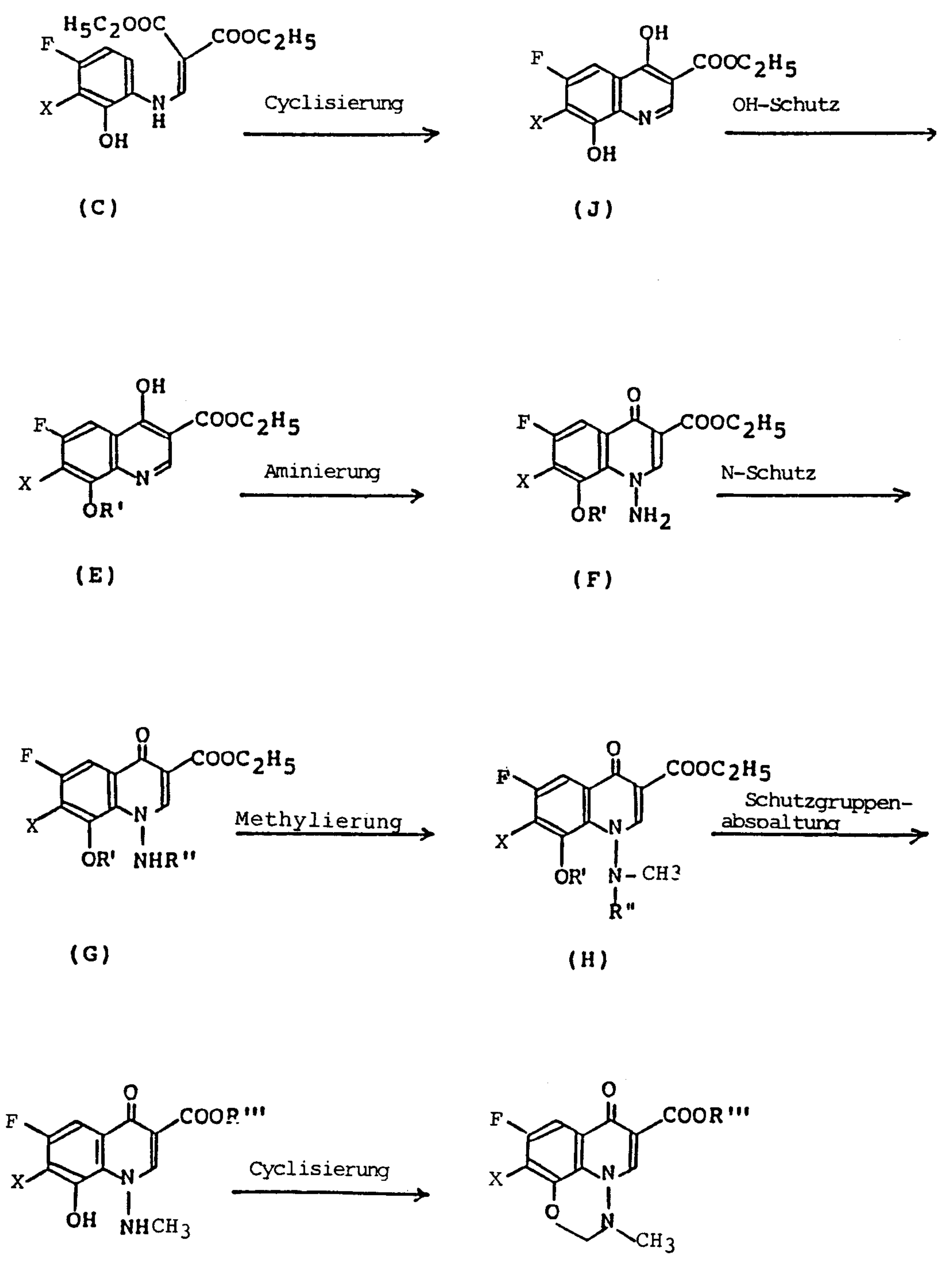


(C) (J)

(E) (F)

(G) (H)

(IVa) (IIa)

worin X die oben gegebene Bedeutung hat; R' ist eine Schutzgruppe, wie z.B. Benzyl, Methoxybenzyl, Methoxymethyl, Methoxyäthoxymethyl, Tetrahydropyranyl, Allyl, t-Butyl, t-Butyldimethylsilyl, Acetyl, Benzoyl und dergleichen; R" ist eine Schutzgruppe, wie Z.B. Formyl, Acetyl, Trifluoracetyl, Benzoyl, Aethoxycarbonyl, 2,2,2-Trichloräthoxycarbonyl, Phenoxycarbonyl, Benzyloxycarbonyl, t-Butoxycarbonyl und dergleichen; und R''' ist ein Wasserstoffatom oder der Aethylrest.

Verbindungen der Formel (III), welche einen Amino- oder Monoalkylamino-Substituenten enthalten, können erwünschtenfalls durch eine Aminoschutzgruppe geschützt sein, wie z.B. durch Formyl, Acetyl, Trifluoracetyl, Benzoyl, Aethoxycarbonyl, 2,2,2-Trichloräthoxycarbonyl, Phenoxycarbonyl, Benzyloxycarbonyl, t-Butoxycarbonyl und dergleichen.

Die Umsetzung der Verbindung der Formel (II) mit dem Amin der Formel (III), das notwendigenfalls geschützt sein kann, kann mit oder ohne Lösungsmittel durchgeführt werden, vorzugsweise bei erhöhter Temperatur bis zur Vollständigen Umsetzung. Die Reaktionstemperatur liegt zweckmässigerweise im Bereiche von etwa 30 °C bis etwa 200 °C, vorzugsweise zwischen 80 °C und 150 °C, damit eine ausreichende Reaktionsgeschwindigkeit erreicht wird.

Die Reaktion wird vorzugsweise in Gegenwart eines Säureacceptors ausgeführt, wie z.B. Triäthylamin, Pyridin, Picolin, N,N-Dimethylanilin, 1,8-Diazabicyclo[5.4.0]undec-7-en, 1,4-Diazabicyclo[2.2.2]octan, Alkalimetallhydroxide, Alkalimetallcarbonate und dergleichen. Wahlweise kann ein Ueberschuss des Amins der Formel (III) als Säureacceptor verwendet werden.

Geeignete Lösungsmittel für diese Umsetzung sind nicht-reaktive Lösungsmittel, wie z.B. Acetonitril, Alkohole, Dimethylsulfoxid, Dimethylformamid, Dimethylacetamid, Pyridin, Picolin, Lutidin, Dimethylpropylharnstoff und dergleichen. Gemische zweier oder mehrerer Lösungsmittel können ebenfalls verwendet werden.

Eine vorhandene Schutzgruppe kann erwünschtenfalls nach der Umsetzung durch an sich bekannte Methoden entfernt werden. Beispielsweise kann die Formylgruppe durch saure oder basische Hydrolyse entfernt werden, vorzugsweise durch basische Hydrolyse; währenddem die Benzyloxycarbonylgruppe durch Hydrogenolyse entfernt werden kann.

Beispiele der Ausgangsverbindungen der Formel (II) sind:
9,10-Difluor-3-methyl-7-oxo-2,3-dihydro-7H-pyrido[3,2,1-ij]-1,3,4-benzoxadiazin-6-carbonsäure,
Aethyl 9,10-difluor-3-methyl-7-oxo-2,3-dihydro-7H-pyrido-[3,2,1-ij]-1,3,4-benzoxadiazin-6-carboxylat,
Benzyl 9,10-difluor-3-methyl-7-oxo-2,3-dihydro-7H-pyrido-[3,2,1-ij]-1,3,4-benzoxadiazin-6-carboxylat,

Das erfindungsgemässe eingesetzte Amin der Formel (III) ist beispielsweise Piperazin, 4-Methylpiperazin, Morpholin, 4-Hydroxypiperidin, 4-Methoxypiperidin, 3-[(Methylamino)-methyl]pyrrolidin, 3-[-(Aethylamino)-methyl]pyrrolidin, 3-Methoxypyrrolidin, 3-Aminopyrrolidin, 3-(Benzyloxycarbonylamino)-pyrrolidin, Imidazol oder 4-Methylimidazol.

Verfahren B:

Die erfindungsgemässen Verbindungen können erhalten werden durch Umsetzung der Verbindung der Formel

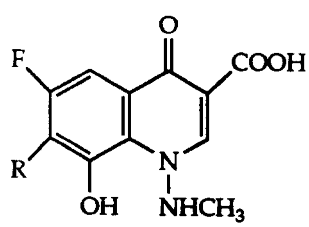

(IV)

in der vorhandene Amino-, Hydroxy- und/oder Carboxygruppen geschützt sein können,
mit Formaldehyd oder Paraformaldehyd, wonach notwendigenfalls eine vorhandene Schutzgruppe abgespalten wird.

Die erfindungsgemäss eingesetzen Ausgangsverbindungen (IV) sind neue Stoffe, welche nach dfem obigen Schema a) oder b) hergestellt werden können oder auch durch Umsetzung einer Verbindung (H) oder (IVa) mit einem Amin der Formel (III).

Diese Umsetzung kann erwünschtenfalls in einem Lösungsmittel ausgeführt werden, z.B. in Dioxan, Tetrahydrofuran, Acetonitril, Chloroform, Dimethylformamid, Dimethylsulfoxid, Dimethylpropylharnstoff, Essigsäure und dergleichen. Gemische von zwei oder mehreren dieser Lösungsmittel können ebenfalls verwendet werden.

Die Umsetzung kann notwendigenfalls in Gegenwart eines sauren Katalysators durchgeführt werden, wie z.B. Essigsäure, Salzsäure, Schwefelsäure, Methansulfonsäure, p-Toluolsulfonsäure, Pyridinium-p-toluolsulfonat, Ferrichlorid, Zinkchlorid, Chlortrimethylsilan, Nafion-H (perfluoriertes Sulfonsäureharz; Aldrich

Chemical Co., Inc.), Amberlyst-15 (stark saures macroretikuläres Harz; Aldrich Chemical Co., Inc.) und dergleichen.

Die Reaktionstemperatur kann innerhalb eines weiten Bereiches variieren. Im allgemeinen wird die Umsetzung bei einer Temperatur zwischen 20 ° C und 150 ° C durchgeführt. Vorzugsweise wird etwa 1 Mol oder ein molarer Ueberschuss des Formaldehyds bzw. Paraformaldehyds pro Mol der Verbindung der Formel (IV) verwendet.

Vorhandene Amino- oder Monoalkylaminoschutzgruppen können erwünschtenfalls nach an sich bekannten Methoden entfernt werden. Beispielsweise kann die Formylgruppe durch saure oder basische Hydrolyse, vorzugsweise durch basische Hydrolyse, entfernt werden; die Benzyloxycarbonylgruppe kann durch Hydrogenolyse entfernt werden.

Beispiele der Ausgangsverbindungen der Formel (IV) sind:
6,7-Difluor-8-hydroxy-1-(methylamino)-4-oxo-1,4-dihydro-3-chinolincarbonsäure,
Aethyl-6,7-difluor-8-hydroxyl-1-(methylamino)-4-oxo-1,4-dihydro-3-chinolincarboxylat,
Benzyl-6,7-difluor-8-hydroxyl-1-(methylamino)-4-oxo-1,4-dihydro-3-chinolincarboxyiat,
6-Fluor-8-hydroxy-7-(1-imidazolyl)-1-(methylamino)-4-oxo-1,4-dihydro-3-chinolincarbonsäure,
Aethyl-6-fluor-8-hydroxy-7-(1-imidazolyl)-1-(methylamino)-4-oxo-1,4-dihydro-3-chinolincarboxylat,
Benzyl-6-fluor-8-hydroxy-7-(1-imidazolyl)-1-(methylamino)-4-oxo-1,4-dihydro-3-chinolincarboxylat,
6-Fluor-8-hydroxy-1-(methylamino)-7-(4-methyl-1-piperazinyl)-4-oxo-1,4-dihydro-3-chinolincarbonsäure,
7-[3-[(Benzyloxycarbonyläthylamino)methyl]-1-pyrrolidinyl]-6-fluor-8-hydroxy-1-(methylamino)-4-oxo-1,4-dihydro-3-chinolincarbonsäure,
7-[3-(Benzyloxycarbonylamino)-1-pyrrolidinyl]-6-fluor-8-hydroxy-1-(methylamino)-4-oxo-1,4-dihydro-3-chinolincarbonsäure,
7-[3-[(Benzyloxycarbonylmethylamino)methyl]-4-methyl-1-pyrrolidinyl]-6-fluor-8-hydroxy-1-(methylamino)-4-oxo-1,4-dihydro-3-chinolincarbonsäure,
7-[3-[(Benzyloxycarbonylamino)methyl]-4-chlor-1-pyrrolidinyl]-6-fluor-8-hydroxy-1-(methylamino)-4-oxo-1,4-dihydro-3-chinolincarbonsäure und dergleichen.

Verfahren C:

Die Verbindung der Formel I worin R die Gruppe

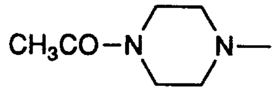

darstellt, kann aus der entsprechenden, unsubstituierten 1-Piperazinylverbindung hergestellt werden, und zwar durch Umsetzung mit einem die Acetylgruppe abgebundenen Mittel, z.B. Essigsäureanhydrid gemäss dem nachstehenden Beispiel 18.

Verfahren D:

Die Verbindung der Formel (I), worin R die Gruppe

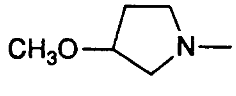

darstellt, kann durch Methylierung der entsprechenden Verbindung worin R die Gruppe

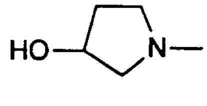

darstellt, erhalten werden.

Diese O-Alkylierung kann durch Umsetzung mit einer Verbindung der allgemeinen Formel

$CH_3Y$ (V)

worin Y eine Abgangsgruppe darstellt,
durchgeführt werden. Abgangsgruppen Y sind beispielsweise Halogenatome wie Chlor, Brom, Jod, Acyloxyreste, wie z.B. Acetoxy; niedere Alkansulfonyloxyreste, wie z.B. Methansulfonyloxy, Arylsulfonyloxyreste, wie z.B. Methansulfonyloxy, Arylsulfonyloxyreste, wie z.B. p-Toluolsulfonyloxy, wahlweise nitrierte Phenoxygruppen wie z.B. Phenoxy, 4-Nitrophenoxy; oder Succinimidooxy oder Phthalimidooxy.

Die Reaktion kann notwendigenfalls in einem Lösungsmittel durchgeführt werden, z.B. in Dimethylformamid, Dimethylacetamid, Dimethylsulfoxid, Dimethyl-n-propylharnstoff, Dioxan, Tetrahydrofuran, Pyridin und dergleichen. Gemische von 2 oder mehreren dieser Lösungsmittel können ebenfalls verwendet werden.

Die Umsetzung wird vorzugsweise in Gegenwart eines Säureacceptors durchgeführt, wie z.B. Triäthylamin, Pyridin, N,N-Dimethylanilin, 1,4-Diazabicyclo[2.2.2]octan, 1,8-Diazabicyclo[5.4.0]undec-7-en, Natriumhydrid, Alkalimetallhydroxide, Alkalimetallcarbonate und dergleichen.

Die Reaktionstemperatur kann innerhalb eines relativ weiten Bereiches variieren. Im allgemeinen wird die Umsetzung bei einer Temperatur zwischen etwa 0 °C und 180 °C durchgeführt, vorzugsweise zwischen 0 °C und 110 °C. Vorzugsweise werden 1 bis 4 Mol, insbesondere 1 bis 2 Mol Methylierungsmittel pro Mol 3-Hydroxy-1-piperidylverbindung eingesetzt. Als Methylierungsmittel wird z.B. Methyljodid verwendet. Vorzugsweise wird ein Protonenakzeptor, wie z.B. ein Alkalimetallhydrid, z.B. Natriumhydrid, zugegeben.

Verfahren E:

Verbindungen der Formel I mit einer freien Aminogruppe können aus der entsprechenden Verbindung der Formel I mit dieser Aminogruppe in geschützter Form hergestellt werden.

Aminoschutzgruppen sind z.B. niederes Alkanoyl, wie z.B. Acetyl; Benzoyl; eine Alkoxycarbonylgruppe, z.B. t-Butoxycarbonyl oder Aethoxycarbonyl; eine substituierte Alkoxygruppe, z.B. Trichloräthoxycarbonyl; Phenoxycarbonyl; Benzyloxycarbonyl; p-Nitrobenzyloxycarbonyl; eine Aralkylgruppe wie z.B. Trityl oder Benzhydryl; oder eine Halogen-alkanoyl-Gruppe wie z.B. Trifluoracetyl.

Die Aminoschutzgruppen sind durch saure Hydrolyse abspaltbar (z.B. t-Butoxycarbonyl oder Trityl) oder auch durch basische Hydrolyse (z.B. Trifluoracetyl). Benzyloxycarbonyl und p-Nitrobenzyloxycarbonyl werden durch Hydrogenolyse abgespalten.

Durch saure Hydrolyse abspaltbare Schutzgruppen werden vorzugsweise mit Hilfe einer niederen Alkancarbonsäure, die gegebenenfalls halogeniert sein kann, entfernt. Insbesondere verwendet man Ameisensäure oder Trifluoressigsaure. Die Temperatur ist in der Regel Raumtemperatur, obwohl auch leicht erhöhte bzw. leicht erniedrigte Temperaturen angewendet werden können, z.B. im Bereich von etwa 0 °C bis +40 °C. Alkalisch abspaltbare Schutzgruppen werden im allgemeinen mit verdünnter wässriger Lauge bei 0 °C bis +30 °C hydrolysiert.

Verfahren F:

Die Verbindung der Formel I worin R die Gruppe

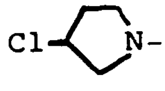

darstellt, kann durch Halogenierung der entsprechenden Verbindung worin R die Gruppe

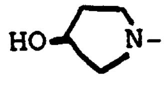

darstellt, erhalten werden. Das Halogenierungsmittel ist vorzugsweise ein Thionylhalogenid, insbesondere Thionylchlorid; oder Phosphortrichlorid, Phosphoroxychlorid oder Phosphorpentachlorid. Die Reaktionstemperatur liegt vorzugsweise zwischen etwa 0 °C und 80 °C Allenfalls vorhandene Carboxygruppen sind vorzugsweise geschützt, z.B. benzyliert, und anschliessend erwünschtenfalls wieder freigesetzt. z.B. durch

Hydrierung (Entfernung von Benzyl).

Verfahren G:

Die Verbindung der Formel I, worin R die Gruppe

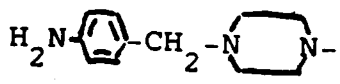

darstellt, kann durch Reduktion der Nitrogruppe der entsprechenden nitro-substituierten Verbindung der Formel I hergestellt werden. Die Reduktion kann durch Hydrierung in Gegenwart eines Edelmetallkatalysators, wie Palladium/Kohle erfolgen. Die Umsetzung wird zweckmässigerweise in Wasser oder einem niederen Alkanol, z.B. Methanol oder Aethanol, durchgeführt, erwünschtenfalls im Gemisch mit anderen darin löslichen Lösungsmitteln. Die Reaktionstemperatur liegt im allgemeinen zwischen etwa 10 °C und etwa 40 °C, vorzugsweise bei etwa Zimmertemperatur.

Verfahren H:

Die Herstellung von pharmazeutisch verträglichen Präparaten der Verbindungen der Formel I oder von Hydraten oder Solvaten dieser Salze kann in an sich bekannter Weise erfolgen; z.B. durch Umsetzung einer Carbonsäure der Formel I mit einer äquivalenten Menge der entsprechenden Base oder, umgekehrt, Umsetzung einer freien Base der Formel I mit einer organischen oder anorganischen Säure. Die Reaktion wird zweckmässig in einem Lösungsmittel durchgeführt, z.B. in Wasser oder in einem organischen Lösungsmittel (z.B. Aethanol, Methanol, Aceton oder dergleichen). Die Temperatur der Salzbildung ist nicht kritisch, sie liegt im allgemeinen bei Raumtemperatur kann aber auch leicht darüber oder darunter, etwa im Bereich von 0 °C bis +50 °C, liegen.

Beispiele von im obigen Verfahren verwendbaren therapeutisch einwandfreien Säuren sind Salzsäure, Bromwasserstoffsäure, Schwefelsäure, Phosphorsäure Salpetersäure, Ameisensäure, Essigsäure, Propionsäure, Bernsteinsäure, Glykolsäure, Milchsäure, Apfelsäure, Weinsäure, Zitronensäure, Ascorbinsäure, Maleinsäure, Hydroxymaleinsäure, Phenylessigsäure, Benzoesäure, 4-Aminobenzoesäure, Anthranilsäure, 4-Hydroxybenzoesäure, Salicylsäure, Aminosalicylsäure, Nicotinsäure, Methansulfonsäure, Aethansulfonsäure, Hydroxyäthansulfonsäure, Benzolsulfonsäure, p-Toluolsulfonsäure, Gluconsäure, Glucuronsäure, Galacturonsäure, Asparaginsäure und Glutaminsäure; Methionin, Tryptophan, Lysin, Arginin und dergleichen.

Die Säureadditionssalze können in die freie Form durch Behandlung mit einer Base, wie z.B. einem Metallhydroxid, Ammoniak oder dergleichen umgewandelt werden.

Die Basensalze der Verbindungen der Formel I können durch Umsetzung einer Verbindung der Formel I mit einer Metallbase oder einem Amin, wie z.B. mit einem Alkali- oder Erdalkalimetallhydroxid oder einem organischen Amin, erhalten werden. Beispiele entsprechender Metallkatione sind Natrium, Kalium, Magnesium, Calcium und dergleichen. Beispiele der Amine sind Diäthanolamin, Dibenzyläthylendiamin, Cholin, Aethylendiamin und dergleichen.

Die Säureadditionssalze oder Basensalze der Verbindungen der Formel I unterscheiden sich von der entsprechenden freien Form in gewissen physikalischen Eigenschaften, wie z.B. durch ihre Löslichkeit in Wasser.

Die Verbindungen der Formel I und ihre pharmazeutisch verträglichen Salze können in unsolvatisierter oder solvatisierter Form, einschliesslich hydratisierter Form, vorliegen. Die Hydratisierung kann automatisch im Zuge des Herstellungsverfahrens erfolgen oder auch als Folge hygroskopischer Eigenschaften eines zunächst wasserfreien Produktes. Zur gezielten Herstellung eines Hydrats kann ein ganz oder teilweise wasserfreies Produkt einer feuchten Atmosphäre, z.B. bei etwa +10 °C bis +40 °C, ausgesetzt werden. Solvate mit pharmazeutisch verträglichen Lösungsmitteln, wie z.B. Aethanol, können beispielsweise während der Kristallisation erhalten werden.

Die erfindungsgemässen Verbindungen besitzen ein breites spektrum antibakterieller Wirkung gegen gram-positive und gram-negative Mikroorganismen und Mycoplasmen; sie können als Mittel für die Behandlung und Prophylaxe von Infektionskrankheiten eingesetzt werden. Die in vitro und in vivo antibakterielle Wirkung der erfindungsgemässen Verbindungen werden wie folgt veranschaulicht:

1. In vitro antibakterielle Wirkung

Die in vitro antibakterielle Wirkung repräsentativer Vertreter der erfindungsgemässen Verbindungen wurde mit Hilfe der Agarverdünnungsmethode getestet [Chemotherapy, 22, 1126 (1974)]. Mindesthemmkonzentrationen (MIC in µg/ml) sind in den Tabellen 1 und 2 aufgeführt.

Tabelle 1

| Aerobischer Mikroorganismus | antimikrobielles Spektrum MIC (µg/ml) Verbindung (Beispiel Nr.) | | | | | | | | | | | |
|---|---|---|---|---|---|---|---|---|---|---|---|---|
| | 2 | 3 | 4 | 5 | 6 | 7 | 10 | 12 | 13 | 14 | 18 | 19 |
| Gram-positive Bakterien | | | | | | | | | | | | |
| Bacillus subtilis PCI 219 | 0.05 | 0.10 | ≤0.0008 | <0.0008 | 0.013 | 0.013 | 0.05 | 0.025 | ≤0.0008 | ≤0.0008 | 0.0065 | 0.025 |
| Staphylococcus aureus FDA 209P JC-1 | 0.39 | 0.39 | 0.20 | 0.10 | 0.05 | 0.05 | 0.20 | 0.05 | 0.0016 | 0.39 | 0.39 | 0.10 |
| Staphylococcus aureus NR 2855 | 0.20 | 3.13 | 0.0065 | 0.05 | 0.20 | 0.10 | 0.39 | 0.39 | ≤0.0008 | 0.20 | 0.39 | 0.10 |
| Staphylococcus aureus Smith | 0.20 | 0.39 | 0.0033 | 0.025 | 0.025 | 0.025 | 0.20 | 0.05 | ≤0.0008 | 0.10 | 0.10 | 0.05 |
| Staphylococcus epidermidis IFO 12993 | 0.39 | 1.56 | 0.20 | 0.10 | 0.20 | 0.10 | 0.39 | 0.20 | 0.025 | 0.39 | 0.39 | 0.10 |
| Staphylococcus epidermidis NR 2942 | 0.39 | 1.56 | 0.0065 | 0.05 | 0.10 | 0.10 | 0.20 | 0.20 | ≤0.0008 | 0.39 | 0.20 | 0.10 |
| Enterococcus faecalis NR 2943 | 3.13 | 12.5 | 12.5 | 3.13 | 1.56 | 1.56 | 3.13 | 3.13 | 3.13 | 12.5 | 3.13 | 1.56 |
| Gram-negative bacteria | | | | | | | | | | | | |
| Alcaligenes faecalis IFO 13111 | 0.39 | 3.13 | 6.25 | 3.13 | 6.25 | 6.25 | 3.13 | 1.56 | 12.5 | 6.25 | 12.5 | 3.13 |
| Citrobacter freundii IFO 12681 | 0.013 | 0.013 | 0.39 | 0.39 | 0.20 | 0.10 | 0.10 | 0.013 | 0.78 | 6.25 | 3.13 | 0.10 |
| Enterobacter aerogenes NR 2945 | 0.013 | 0.013 | 0.0065 | 0.20 | 0.05 | 0.05 | 0.10 | 0.0033 | 0.10 | 3.13 | 0.78 | 0.10 |
| Enterobacter cloacae NR 2946 | 0.025 | 0.025 | 0.013 | 0.39 | 0.10 | 0.05 | 0.10 | 0.013 | 0.20 | 6.25 | 1.56 | 0.20 |
| Escherichia coli NIHJ JC-2 | 0.025 | 0.025 | 0.10 | 0.39 | 0.10 | 0.05 | 0.10 | 0.013 | 0.10 | 6.25 | 1.56 | 0.20 |
| Escherichia coli NR 2630 | 0.013 | 0.025 | ≤0.0008 | - | 0.025 | 0.013 | 0.025 | 0.0065 | ≤0.0008 | 0.39 | 0.20 | 0.05 |
| Klebsiella pneumoniae FDA PCI 602 | 0.10 | 0.10 | 3.13 | 0.78 | 0.78 | 0.39 | 0.39 | 0.10 | 3.13 | 25 | 6.25 | 0.39 |
| Bordetella bronchiseptica ATCC 4617 | 0.20 | 6.25 | 1.56 | 0.78 | 6.25 | 3.13 | 1.56 | 1.56 | 1.56 | 6.25 | 3.13 | 0.78 |
| Proteus rettgeri ATCC 14505 | 0.20 | 0.10 | 1.56 | 0.78 | 1.56 | 0.39 | 0.78 | 0.10 | 6.25 | 25 | 6.25 | 1.56 |
| Proteus vulgaris OX19 ATCC 6898 | 0.013 | 0.013 | ≤0.0008 | 0.05 | 0.025 | 0.025 | 0.0065 | 0.0065 | ≤0.0008 | 3.13 | 0.39 | 0.10 |
| Pseudomonas aeruginosa A3 | 0.20 | 0.10 | 6.25 | 1.56 | 0.39 | 0.78 | 1.56 | 0.05 | 6.25 | 25 | 6.25 | 0.78 |
| Pseudomonas aeruginosa NR 2950 | 0.78 | 0.78 | 12.5 | 6.25 | 6.25 | 6.25 | 6.25 | 0.20 | 25 | 50 | 25 | 12.5 |
| Pseudomonas stutzeri IFO 12695 | 0.025 | 0.025 | ≤0.0008 | 0.10 | 0.025 | 0.025 | 0.10 | 0.0033 | 0.013 | 6.25 | - | 0.20 |
| Serratia marcescens IFO 12648 | 0.10 | 0.10 | 0.78 | 0.39 | 0.78 | 0.39 | 0.78 | 0.05 | 0.78 | 12.5 | 3.13 | 0.78 |
| Salmonella typhimurium IFO 12529 | 0.025 | 0.013 | 0.0033 | 0.10 | 0.05 | 0.025 | 0.10 | 0.0065 | 0.20 | 6.25 | 0.78 | 0.10 |

## Tabelle 1 (Fortsetzung)

| Aerobischer Mikroorganismus | antimikrobielles Spektrum MIC (µg/ml) Verbindung (Beispiel Nr.) 20 | 22 | 25 |
|---|---|---|---|
| **Gram-positive Bakterien** | | | |
| Bacillus subtilis PCI 219 | 0.025 | 0.20 | 0.0065 |
| Staphylococcus aureus FDA 209P JC-1 | 0.013 | 0.39 | 0.39 |
| Staphylococcus aureus NR 2855 | 0.20 | 0.78 | 0.10 |
| Staphylococcus aureus Smith | 0.025 | 0.39 | 0.05 |
| Staphylococcus epidermidis IFO 12993 | 0.10 | 0.39 | 0.39 |
| Staphylococcus epidermidis NR 2942 | 0.10 | 0.39 | 0.20 |
| Enterococcus faecalis NR 2943 | 1.56 | 12.5 | 3.13 |
| **Gram-negative bacteria** | | | |
| Alcaligenes faecalis IFO 13111 | 6.25 | 3.13 | 6.25 |
| Citrobacter freundii IFO 12681 | 0.10 | 0.10 | 1.56 |
| Enterobacter aerogenes NR 2945 | 0.025 | 0.10 | 0.78 |
| Enterobacter cloacae NR 2946 | 0.10 | 0.05 | 0.78 |
| Escherichia coli NIHJ JC-2 | 0.10 | 0.10 | 1.56 |
| Escherichia coli NR 2630 | 0.025 | 0.0065 | 0.39 |
| Klebsiella pneumoniae FDA PCI 602 | 0.78 | 0.39 | 3.13 |
| Bordetella bronchiseptica ATCC 4617 | 3.13 | 3.13 | 3.13 |
| Proteus rettgeri ATCC 14505 | 0.78 | 1.56 | 3.13 |
| Proteus vulgaris OX19 ATCC 6898 | 0.013 | 0.013 | 0.20 |
| Pseudomonas aeruginosa A3 | 0.20 | 3.13 | 3.13 |
| Pseudomonas aeruginosa NR 2950 | 1.56 | 6.25 | 25 |
| Pseudomonas stutzeri IFO 12695 | 0.013 | 0.0033 | 1.56 |
| Serratia marcescens IFO 12648 | 0.39 | 0.78 | 3.13 |
| Salmonella typhimurium IFO 12529 | 0.025 | 0.05 | 1.56 |

Tabelle 2

| Antibakterielles Spektrum MIC (µg/ml) | Verbindung (Beispiel Nr. ) | |
|---|---|---|
| | 2 | 12 |
| Bacteroides fragilis ATCC 23745 | 0.78 | 0.20 |
| Bacteroides fragilis NR 2579 | 3.13 | 1.56 |
| Bacteroides fragilis NR 2582 | 0.78 | 0.39 |
| Bacteroides fragilis NR 2583 | 0.39 | 0.10 |
| Bacteroides fragilis NR 2584 | 0.78 | 0.78 |
| Bacteroides distasonis NR 2578 | 0.78 | 0.78 |
| Bacteroides thetaiotaomicron NR 2588 | 1.56 | 0.78 |
| Bifidobacterium adolescentis ATCC 15703 | 0.39 | 0.10 |
| Clostridium botulinum NR 2611 | 0.10 | 0.013 |
| Clostridium perfringens NR 2612 | 0.39 | 0.10 |
| Clostridium moniliforme ATCC 25546 | 0.78 | 0.10 |
| Fusobacterium varium ATCC 8501 | 12.5 | - |
| Peptococcus prevotii ATCC 9321 | 1.56 | 0.39 |
| Peptococcus variabilis ATCC 14955 | 0.78 | 0.20 |
| Peptostreptococcus anaerobius NR 2743 | 0.39 | 0.013 |
| Propionibacterium acnes ATCC 11828 | 0.78 | 0.78 |
| Mycoplasma | | |
| Mycoplasma hominis NR 2952 | 0.10 | 0.10 |

2. In vivo therapeutische Wirksamkeit

Die in vivo antibakterielle Wirksamkeit der Verbindungen der Beispiele 5, 30, 65 und 66 wurde gegen Lethalinfektionen von Escherichia coli ML4707, Pseudomonas aeruginosa 4au542 und Streptococcus

pneumoniae 6-001 gemessen. ICR-Mäuse von etwa 20 g wurden mit einer entsprechenden bakteriellen Suspension intraperitoneal infiziert. Die Testverbindungen wurden oral oder subkutan zum Zeitpunkt der Infektion verabreicht. Die Mortalität wurde 5 Tage beobachtet. Die 50% wirksame Dosis ($ED_{50}$, mg/kg). welche 50% der Tiere gegen Tod durch Infektion schützt, ist aus Tabelle 3 ersichtlich.

Tabelle 3

| In vivo antibakterielle Wirkung gegen systemische Infektionen an der Maus ($ED_{50}$, mg/kg) | | | | |
|---|---|---|---|---|
| Verbindung | Bakterium | | | |
| | Escherichia coli ML4707 | | Pseudomonas aeruginosa 4au542 | Streptococcus pneumoniae 6-001 |
| | s.c. | p.o. | p.o. | p.o. |
| Beispiel 2 | 0.06 | 0.11 | 13.4 | 10.3 |
| Beispiel 12 | 0.10 | 0.62 | 57.0 | 65.9 |

3. Akute Toxizität

Die $LD_{50}$-Werte der Verbindungen aus den Beispielen 2, 3, 6, 7, 12 und 22 liegen alle über 2000 mg/kg (akute Toxizität nach oraler Verabreichung an ICR-Mäusen).

Die erfindungsgemässen Verbindungen besitzen ein breites antimikrobielles Spektum gegen gram-positive und gram-negative Bakterien und Mycoplasmen,, insbesondere gegen Erreger, welche gegen verschiedenen Antibiotika resistent sind. wie z.B. Penicilline, Cephalosporine, Aminoglycoside, Tetracycline und dergleichen.

Die erfindungsgemässen Verbindungen besitzen ferner eine niedere Toxizität in Kombination mit kräftiger und breiter antimikrobieller Wirksamkeit. Die Schutzwirkung der erfindungsgemässen Verbindungen bei systemischen bakteriellen Infektionen an Mäusen ist grösser als diejenige kommerziell erhältlicher synthetischer antibakterieller Mittel. Die erfindungsgemässen Verbindungen können demgemäss für die Verhinderung oder Behandlung von Krankheiten, welche durch gram-positive und gram-negative Bakterien verursacht sind, sowie auch gegen bakterioiden Mikroorganismen in der Human- und Tier-Medizin, wirksam verwendet werden .

Beispielsweise können Krankheiten behandelt und/oder verhütet werden, welche durch die folgenden Mikroorganismen bzw. Mischungen der folgenden Mikroorganismen verursacht werden: Staphylococcus, Streptococcus, Aerococcus, Enterococcus, Micrococcus, Lactobacillus, Bifidobacterium, Clostridium, Eubacterium, Peptococcus, Peptostreptococcus, Propionibacterium, Escherichia, Citrobacter, Campylobacter, Enterobacter, Klebsiella, Proteus, Pseudomonas, Serratia, Salmonella, Shigella, Vibrio, Aeromonas, Haemophilus, Neisseria, Acinetobacter, Alcaligenes, Bordetella, Bacteroides, Fusobacterium, Mycoplasma sowie andere Mikroorganismen.

Die Erfindung betrifft ferner pharmazeutische Präparate enthaltend eine oder mehrere der erfindungsgemässen Verbindungen.

Die erfindungsgemässen Erfindungen können oral oder nicht-oral (parenteral) an Menschen oder Tieren durch verschiedene herkömmliche Administrierungsmethoden verabreicht werden.

Die erfindungsgemässen Verbindungen können als solche oder auch in Formulierung mit Hilfsstoffen, flüssigen Verdünnungsmitteln, Bindemitteln, Gleitmitteln, Feuchthaltemitteln, etc. eingesetzt werden, z.B. in der Form allgemeiner Arzneimittel, wie z.B. Tabletten, Granulate, zuckerbeschichtete Tabletten, Pulver, Kapseln, Gele, Trockensirupe, Sirupe, Ampullen, Suspensionen, Flüssigkeiten, Emulsionen, Salben, Pasten, Cremes, Suppositorien und dergleichen.

Ferner können lösungsverzögernde, absorptionsbeschleunigende, oberflächenaktive Agenzien und dergleichen sowie auch andere Zusatzmittel verwendet werden, d.h. jegliche Präparateform, die pharmazeutisch verträglich ist, kann verwendet werden.

Die erfindungsgemässen Verbindungen können allein verwendet werden oder auch als Gemische von zwei oder mehreren Verbindungstypen; mengenmässig können die Verbindungen zu etwa 0,1 bis 99,5%, vorzugsweise 0,5 bis 95%, auf der Basis des fertigen Arzneimittels eingesetzt werden.

Die Tagesdosis einer erfindungsgemässen Verbindung kann nach dem Bedarf des zu behandelnden Menschen bzw. Tieres sowie in Abhängigkeit von dessen Gewicht sowie des zu behandelnden Zustandes

variieren, sie liegt jedoch im allgemeinem im Bereiche von 0,5 bis 500 mg pro kg Gewicht, vorzugsweise bei etwa 1 bis 300 mg/kg.

Die folgenden Beispiele veranschaulichen die Herstellung der erfindungsgemässen Verbindungen.

Herstellung von Ausgangsverbindungen

Bezugsbeispiele.

Herstellung von Diäthyl N-(3,4-difluor-2-hydroxyphenyl)aminomethylenmalonat

(a) Eine Lösung von 500 mg 2,3-Difluor-6-nitrophenol in 7 ml Methanol wird über 60 mg 5% Pd/C 6 Stunden hydriert. Das Reaktionsgemisch wird in einer Stickstoffatmosphäre filtriert und das Filtrat unter vermindertem Druck eingedampft. Man erhält rohes 2-Amino-5,6-difluorphenol.

(b) Ein Gemisch von 414 mg 2-Amino-5,6-difluorphenol und 618 mg Diäthyläthoxymethylenmalonat wird bei 130 °C in einer Stickstoffatmosphäre 5 Minuten erhitzt. Der erhaltene kristalline Rückstand wird mit Aethanol verrieben und filtriert. Man erhält Diäthyl-N-(3,4-difluor-2-hydroxyphenyl)-aminomethylenmalonatvom Smp. 178-180 °C; MS m/z 315 ($M^+$). Die Mutterlauge wird an Silicagel chromatographiert mit Chloroform/Aceton (20:1) als Eluierungsmittel, wobei eine zusätzliche Kristallernte erhalten wird.

Herstellung von Aethyl-8-benzyloxy-6,7-difluor-4-hydroxy-3-chinolincarboxylat (Syntheseweg 1)

(c) Ein Gemisch von Diäthyl-N-(3,4-difluor-2-hydroxyphenyl)-aminomethylenmalonat und 70 mg wasserfreiem Natriumcarbonat in 1,5 ml trockenem Dimethylformamid wird mit 30 µl Benzylbromid versetzt. Das Gemisch wird 2 Stunden bei Zimmertemperatur gerührt. Nach Verdampfen des Lösungsmittels unter vermindertem Druck wird der Rückstand in Methylchlorid gelöst und der erhaltene Niederschlag abfiltriert. Das Filtrat wird mit Wasser gewaschen, über wasserfreiem Natriumsulfat getrocknet und eingedampft. Der kristalline Rückstand wird mit n-Hexan gewaschen und aus Methanol umkristallisiert. Man erhält Diäthyl-N-(2-benzyloxy-3,4-difluorphenyl)-aminomethylenmalonat vom Smp. 87 °C; MS m/z 405 ($M^+$).

(d) Eine Lösung von 280 mg des obigen Malonats in 2,8 ml Diphenyläther wird bei 250 °C 30 Minuten in einer Stickstoffatmosphäre erhitzt. Nach Abkühlung des Reaktionsgemisches wird das erhaltene Aethanol unter vermindertem Druck entfernt. Die zurückbleibende dunkelbraune Lösung wird an 10 g Kieselgel chromatographiert und nacheinander mit Benzol, Methylenchlorid und Methylenchlorid/Aceton (30:1) eluiert. Die reinen Fraktionen werden vereinigt und das Lösungsmittel unter Vermindertem Druck abgedampft` Der kristalline Rückstand wird mit einem Gemisch von n-Hexan und Aethylacetat gewaschen; man erhält Aethyl-8-benzyloxy-6,7-difluor-4-hydroxy-3-chinolincarboxylat, das nach dem Umkristallisieren aus Methanol bei 200-201 °C schmilzt; MS m/z 359 ($M^+$).

Herstellung von Aethyl-8-benzyloxy-6,7-difluor-4-hydroxy-3-chinolincarboxylat (Syntheseweg 2)

Eine Lösung von 300 mg Aethyl-6,7-difluor-4,8-dihydroxy-3-chinolincarboxylat in 6 ml trockenem Dimethylformamid wird nacheinander mit 308 mg wasserfreiem Kaliumcarbonat und 145 µl Benzylchlorid Versetzt. Das Gemisch wird 11 Stunden bei 55-65 °C gerührt. Das Reaktionsgemisch wird mit 30 ml Wasser verdünnt und mit Chloroform extrahiert. Der Extrakt wird über wasserfreiem Natriumsulfat getrocknet und unter vermindertem Druck konzentriert. Der Rückstand wird an 7 g Kieselgel chromatographiert mit Aceton/Chloroform (1:20) als Eluierungsmittel. Man erhält Aethyl-8-benzyloxy-6,7-difluor-4-hydroxy-3-chinolincarboxylat, das nach dem Umkristallisieren aus Methanol bei 200-201 °C schmilzt; MS m/z 359 ($M^+$).

Herstellung von Aethyl-8-benzyloxy-6,7-difluor-1-(formylmethylamino)-4-oxo-1,4-dihydro-3-chinolincarboxylat

(e) Ein Gemisch von 410 mg Aethyl-8-benzyloxy-6,7-difluor-4-hydroxy-3-chinolincarboxylat und 315 mg wasserfreiem Kaliumcarbonat in 10 ml trockenem Dimethylformamid wird 3 Stunden bei Zimmertemperatur gerührt und anschliessend mit 260 mg O-(2,4-Dinitrophenyl)hydroxylamin versetzt. Das Reaktionsgemisch wird bei Zimmertemperatur weitere 6,5 Stunden gerührt. Nach der Entfernung des Lösungsmittels unter vermindertem Druck werden 12 ml Wasser hinzugefügt und das Gemisch bei Zimmertemperatur 3 Stunden gerührt. Der erhaltene Niederschlag wird abfiltriert und nacheinander mit kaltem Wasser und Aether gewaschen. Man erhält 1-Amino-8-benzyloxy-6,7-difluor-4-oxo-1,4-dihydro -3-chinolincarboxylat, das nach dem Umkristallisieren aus methanol bei 143-144 °C schmilzt; MS m/z 374 ($M^+$).

(f) 1,51 ml Essigsäureanhydrid werden bei 0 °C mit 0,60 ml 98%iger Ameisensäure versetzt. Das Gemisch wird 15 Minuten bei 0 °C und 15 Minuten bei 50 °C gerührt und anschliessend auf 0 °C abgekühlt. Diese Lösung wird tropfenweise mit einer Lösung von 400 mg des obigen Amins in 2,1 ml 98%iger Ameisensäure Versetzt. Das Gemisch wird bei Zimmertemperatur 2 Tage gerührt. Das Reaktionsgemisch wird unter vermindertem Druck eingedampft und der kristalline Rückstand aus Aethanol umkristallisiert. Man erhält Aethyl-8-benzyloxy-6,7-difluor-1-(formylamino)-4-oxo-1,4 -dihydro-3-chinolincarboxylat vom Smp. 188-190 °C; MS m/z 402 ($M^+$).

(g) Ein Gemisch von 400 mg des obigen Formamids, 275 mg wasserfreiem Kaliumcarbonat und 17 ml wasserfreiem Dimethylformamid wird 1,5 Stunden bei Zimmertemperatur gerührt. Das Reaktionsgemisch wird mit 0,19 ml Methyljodid versetzt und 2,5 Stunden weitergerührt. Das Lösungsmittel wird unter vermindertem Druck entfernt und der Rückstand zwischen Chloroform und Wasser verteilt. Die organischen Phasen werden über wasserfreiem Natriumsulfat getrocknet und unter vermindertem Druck zur Trockene eingedampft. Der Rückstand wird aus Aethanol umkristallisiert; man erhält Aethyl-8-benzyloxy-6,7-difluor-1-(formylmethylamino)-4-oxo -1,4-dihydro-3-chinolincarboxylat vom Smp. 180-181 °C; MS m/z 416 ($M^+$).

Herstellung von 6,7-Difluor-8-hydroxy-1-(methylamino)-4-oxo-1,4-dihydro-3-chinolincarbonsäure

(h) 330 mg Aethyl-8-benzyloxy-6,7-difluor-1-(formylmethylamino)-4-oxo-1,4-dihydro-3-chinolincarboxylat werden über 50 mg 5% Pd/C in 14 ml Chloroform 4 Stunden hydriert. Das Reaktionsgemisch wird mit 14 ml Methanol verdünnt und filtriert. Der Filterkuchen wird mit Chloroform/Methanol 1:1 gewaschen. Die vereinigten Filtrate werden eingedampft und der Rückstand aus Aethanol umkristallisiert. Man erhält Aethyl-6,7-difluor-1-(formylmethylamino)-8-hydroxy-4-oxo -1,4-dihydro-3-chinolincarboxylat vom Smp. 221-225 °C (Zers.); MS m/z 326 ($M^+$).

(i) Ein Gemisch von 210 mg des obigen Esters und 5,2 ml 0,5N wässriger Natriumhydroxidlösung wird bei 100 °C 2 Stunden in einer Stickstoffatmosphäre erhitzt. Das Reaktionsgemisch wird mit 0,16 ml Essigsäure angesäuert. Der erhaltene Niederschlag wird abfiltriert, mit Wasser gewaschen und unter vermindertem Druck getrocknet. Man erhält 6,7-Difluor-8-hydroxy-1-(methylamino)-4-oxo-1,4-dihydro -3-chinolincarbonsäure, die nach dem Umkristallisieren aus Aethanol bei 248-250 °C (Zers.) schmilzt; MS m/z 270 ($M^+$).

Von den nachstehenden Beispielen 1-32 erläutern die Beispiele 1-14 und 18-32 die erfindungsgemässen Verbindungen bzw. deren Herstellung. Die Beispiele 15-17 dienen der Vollständigkeit der Offenbarung.

Beispiel 1

Herstellung von 9,10-Difluor-3-methyl-7-oxo-2,3-dihydro-7H-pyrido[3,2,1-ij]-1,3,4-benzoxadiazin-6-carbonsäure

Ein Gemisch von 105 mg 6,7-Difluor-8-hydroxy-1-(methylamino)-4-oxo-1,4-dihydro -3-chinolincarbonsäure (aus Bezugsbeispiel (i)), 150 mg Paraformaldehyd und 5 ml trockenem Dioxan wird bei 100 °C 3 Stunden in einer Stickstoffatmosphäre erhitzt. Nach Entfernung des Lösungsmittels unter vermindertem Druck werden 20 ml Dimethylformamid hinzugefügt, das Gemisch wird 20 Minuten gerührt und anschliessend filtriert. Der Filterkuchen wird mit Dimethylformamid gewaschen und das Filtrat unter vermindertem Druck eingedampft. Der Rückstand wird mit Wasser verrieben und filtriert. Man erhält 9,10-Difluor-3-methyl-7-oxo-2,3-dihydro-7H-pyrido[3,2,1-ij] -1,3,4-benzoxadiazin-6-carbonsäure, die nach dem Umkristallisieren aus Dimethylformamid bei 290-292 °C unter Zersetzung schmilzt; MS m/z 282 ($M^+$).

Beispiel 2

Herstellung von 9-Fluor-3-methyl-10-(4-methyl-1-piperazinyl)-7-oxo-2,3-dihydro-7H-pyrido[3,2,1-ij]-1,3,4-benzoxadiazin-6-carbonsäure

Ein Gemisch von 30 mg 9,10-Difluor-3-methyl-7-oxo-2,3-dihydro-7H-pyrido[3,2,1-ij] -1,3,4-benzoxadiazin-6-carbonsäure (aus Beispiel 1), 47 µl N-Methylpiperazin und 3 ml trockenem Pyridin werden bei 100-110 °C 9 Stunden in einer Stickstoffatmosphäre erhitzt. Das Pyridin wird unter vermindertem Druck entfernt und der Rückstand aus Methanol umkristallisiert. Man erhält 9-Fluor-3-methyl-10-(4-methyl-1-piperazinyl)-7-oxo-2,3-dihydro -7H-pyrido[3,2,1-ij]-1,3,4-benzoxadiazin-6-carbonsäure vom Smp. 268-269 °C (Zers.); MS m/z 362 ($M^+$).

Die nachstehenden Verbindungen sind in Analogie zu Beispiel 2 erhältlich:

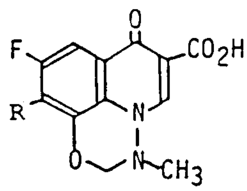

| Beispiel No. | R | Schmelzpunkt °C | Lösungsmittel für die Umkristallisation | MS m/z |
|---|---|---|---|---|
| 3 | 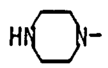 | 240∿245 (dec.) | MeOH | 349 ($MH^+$)* |
| 4 | 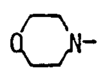 | >300 | $EtOH/CHCl_3$ | 349 ($M^+$) |
| 5 | 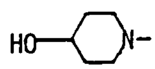 | 256∿258 | $MeOH/CHCl_3$ | 363 ($M^+$) |
| 6 | 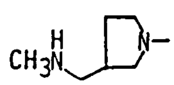 | 242∿244 (dec.) | DMF | 377 ($MH^+$)* |
| 7 | 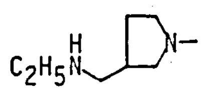 | 251∿252 (dec.) | EtOH | 391 ($MH^+$)* |
| 8 | 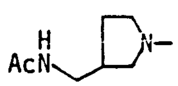 | 239∿241 (dec.) | EtOH | 405 ($MH^+$)* |
| 9 | 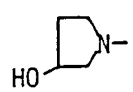 | 284∿286 (dec.) | DMF | 350 ($MH^+$)* |
| 10 | 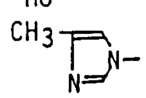 | 280∿284 (dec.) | $MeOH/CHCl_3/Et_2O$ | 345 ($MH^+$)* |

* FAB-MS

Beispiel 11

Herstellung von 10-[3-(Benzyloxycarbonylamino)-1-pyrrolidinyl]-9-fluor-3-methyl-7-oxo-2,3-dihydro-7H-pyrido[3,2,1-ij]-1,3,4-benzoxadiazin-6-carbonsäure

Ein Gemisch von 28 mg 9,10-Difluor-3-methyl-7-oxo-2,3-dihydro-7H-pyrido[3,2,1-ij] -1,3,4-benzoxadiazin-6-carbonsäure (aus Beispiel 1), 94 mg 3-(Benzyloxycarbonylamino)-pyrrolidin und 3 ml trockenem Pyridin werden auf 100 °C 3 Stunden in einer Stickstoffatmosphäre erhitzt. Das Pyridin wird unter vermindertem Druck entfernt und der Rückstand aus Methanol umkristallisiert. Man erhält 10-[3-(Benzyloxycarbonylamino)-1-pyrrolidinyl]-9-fluor -3-methyl-7-oxo-2,3-dihydro-7H-pyrido[3,2,1-ij] -1,3,4-benzoxadiazin-6-carbonsäure vom Smp. 227-230 °C; MS m/z 482 ($M^+$).

Beispiel 12

Herstellung von 10-(3-Amino-1-pyrrolidinyl)-9-fluor-3-methyl-7-oxo-2,3-dihydro-7H-pyrido[3,2,1-ij]-1,3,4-benzoxadiazin-6-carbonsäure

30 mg 10-[3-(Benzyloxycarbonylamino)-1-pyrrolidinyl]-9-fluor -3-methyl-7-oxo-2,3-dihydro-7H-pyrido-[3,2,1-ij] -1,3,4-benzoxadiazin-6-carbonsäure (aus Beispiel 11) werden über 10 mg 5% Pd/C in 2 ml Dimethylformamid 4,5 Stunden hydriert. Nach Entfernung des Katalysators durch Filtrieren wird das Filtrat unter vermindertem Druck eingedampft. Der Rückstand wird aus Methanol umkristallisiert. Man erhält 10-(3-Amino-1-pyrrolidinyl)-9-fluor-3-methyl-7-oxo-2,3 -dihydro-7H-pyrido[3,2,1-ij]-1,3,4-benzoxadiazin-6-carbonsäure vom Smp. 230-234 °C (Zers.); MS m/z 348 ($M^+$).

Beispiel 13

Herstellung von 9-Fluor-10-(3-methoxy-1-pyrrolidinyl)-3-methyl-7-oxo-2,3-dihydro-7H-pyrido[3,2,1-ij]-1,3,4-benzoxadiazin-6-carbonsäure

Eine Aufschlämmung von 100 mg 9-Fluor-10-(3-hydroxy-1-pyrrolidinyl)-3-methyl-7-oxo-2,3 -dihydro-7H-pyrido-[3,2,1-ij]-1,3,4-benzoxadiazin-6-carbonsäure (aus Beispiel 9) in 10 ml trockenem Dimethylformamid wird mit 30 mg 60%igem Natriumhydrid (in Oel) und 40 µl Methyljodid versetzt. Das Gemisch wird bei Zimmertemperatur 2 Stunden gerührt, anschliessend mit weiteren 30 mg 60%igem Natriumhydrid in Oel und 40 µl Methyljodid versetzt und bei 45 °C 3 Stunden gerührt. Das Lösungsmittel wird unter vermindertem Druck eingedampft. Der Rückstand wird mit 2 ml kaltem Wasser und 2,3 ml 0,5N wässriger Natriumhydroxidlösung versetzt und die erhaltene Suspension auf 100 °C 3 Stunden erhitzt. Das Reaktionsgemisch wird auf Zimmertemperatur abgekühlt, mit Essigsäure neutralisiert und mit Wasser verdünnt. Das Gemisch wird mit Chloroform extrahiert und das Extrakt mit Wasser gewaschen, über wasserfreiem Natriumsulfat getrocknet und unter vermindertem Druck eingedampft. Der kristalline Rückstand wird an Kieselgel mit Aceton/Chloroform 1:9 als Eluierungsmittel chromatographiert. Nach dem Umkristallisieren aus Methanol erhält man 9-Fluor-10-(3-methoxy-1-pyrrolidinyl)-3-methyl-7-oxo-2,3 -dihydro-7H-pyrido-[3,2,1-ij]-1,3,4-benzoxadiazin-6-carbonsäure vom Smp. 233-234 °C; FAB-MS m/z 364 ($MH^+$).

Beispiel 14

Herstellung von 9-Fluor-10-(4-methoxy-1-piperidyl)-3-methyl-7-oxo-2,3-dihydro-7H-pyrido[3,2,1-ij]-1,3,4-benzoxadiazin-6-carbonsäure

9-Fluor-10-(4-methoxy-1-piperidyl)-3-methyl-7-oxo-2,3 -dihydro-7H-pyrido[3,2,1-ij]-1,3,4-benzoxadiazin -6-carbonsäure wird hergestellt aus 9-Fluor-10-(4-hydroxy-1-piperidyl)-3-methyl-7-oxo-2,3-dihydro -7H-pyrido[3,2,1-ij]-1,3,4-benzoxadiazin-6-carbonsäure (aus Beispiel 5 ) in Analogie zu Beispiel 13. Die Verbindung wird aus Chloroform/n-Hexan umkristallisiert und schmilzt dann bei 229-233 °C (Zers.); MS m/z 377 ($M^+$).

Beispiel 15

Herstellung von 9-Fluor-3-methyl-7-oxo-10-[4-(2-oxo-n-propyl)-1-piperazinyl]-2,3-dihydro-7H-pyrido[3,2,1-ij]-1,3,4-benzoxadiazin-6-carbonsäure

Ein Gemisch von 50 mg 9-Fluor-3-methyl-7-oxo-10-(1-piperazinyl)-2,3-dihydro-7H -pyrido[3,2,1-ij]-1,3,4-benzoxadiazin-6-carbonsäure (aus Beispiel 3), 17 µl Chloraceton, 40 µl Triäthylamin und 1 ml trockenem Dimethylformamid wird 3,5 Stunden auf 80 °C erhitzt. Die flüchtigen Komponenten werden anschliessend unter vermindertem Druck entfernt und der Rückstand in Wasser aufgeschlämmt. Der erhaltene Niederschlag wird durch Filtrieren isoliert und aus einem Gemisch von Methylenchlorid und Methanol umkristallisiert. Man erhält 9-Fluor-3-methyl-7-oxo-10-[4-(2-oxo-n-propyl) -1-piperazinyl]-2,3-dihydro-7H-pyrido[3,2,1-ij]-1-3,4 -benzoxadiazin-6-carbonsäure vom Smp. 225-229 °C (Zers.); FAB-MS m/z 405 ($MH^+$).

Die nachstehende Verbindung (Beispiel 16) ist in Analogie zu Beispiel 15 erhältlich.

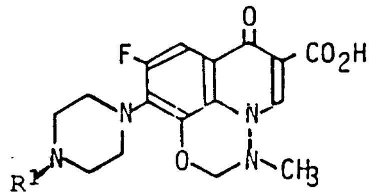

| Beispiel No. | $R^1$ | Schmelzpunkt °C | Lösungsmittel für die Umkristallisation | FAB-MS m/z |
|---|---|---|---|---|
| 16 | 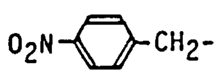 | 275~276 (dec.) | EtOH | 484 ($MH^+$) |

Beispiel 17

Herstellung von 10-[4-(3-Carboxypropionyl)-1-piperazinyl]-9-fluor-3-methyl-7-oxo-2,3-dihydro-7H-pyrido-[3,2,1-ij]-1,3,4-benzoxadiazin-6-carbonsäure

Ein Gemisch von 50 mg 9-Fluor-3-methyl-7-oxo-10-(1-piperazinyl)-2,3-dihydro -7H-pyrido[3,2,1-ij]-1,3,4-benzoxadiazin-6-carbonsäure (aus Beispiel 3 ), 21,6 mg Bernsteinsäureanhydrid, 40 µl Triäthylamin und 4 ml trockenem Dimethylformamid wird 2 Stunden auf 80 °C erhitzt. Das Lösungsmittel wird unter vermindertem Druck abgedampft und der Rückstand in Wasser aufgeschlämmt. Der Niederschlag wird abfiltriert und aus einem Gemisch von Methylenchlorid und Methanol umkristallisiert. Man erhält 10-[4-(3-Carboxypropionyl)-1-piperazinyl]-9-fluor-3-methyl -7-oxo-2,3-dihydro-7H-pyrido[3,2,1-ij]-1,3,4-benzoxadiazin -6-carbonsäure vom Smp. 257-259 °C (Zers.); FAB-MS m/z 449 ($MH^+$).

Beispiel 18

Herstellung von 10-(4-Acetyl-1-piperazinyl)-9-fluor-3-methyl-7-oxo-2,3-dihydro-7H-pyrido[3,2,1-ij]-1,3,4-benzoxadiazin-6-carbonsäure

10-(4-Acetyl-1-piperazinyl)-9-fluor-3-methyl-7-oxo-2,3 -dihydro-7H-pyrido[3,2,1-ij]-1,3,4-benzoxadiazin -6-carbonsäure wird hergestellt aus 9-Fluor-3-methyl-7-oxo-10-(1-piperazinyl)-2,3-dihydro-7H -pyrido[3,2,1-ij]-1,3,4-benzoxadiazin-6-carbonsäure (aus Beispiel 3) und Essigsäureanhydrid in Analogie zu Beispiel 17. Nach dem Umkristallisieren aus Methylenchlorid/Methanol schmilzt die Verbindung bei 294-296 °C (Zers.); FAB-MS m/z 391 ($MH^+$). -6-carboxylat vom Smp. 260-263 °C (Zers.); $^1H$-NMR ($D_2O$) $\delta$: 2,98 (3H,s), 3,05 (4H,m), 3.39 (4H,m), 3,84 (2H,s), 5,18 (2H,s), 7,55 (1H,d,J = 13,4Hz), 8,34 (1H,s).

Beispiel 19

Herstellung von 10-[4-(4-Aminobenzyl)-1-piperazinyl]-9-fluor-3-methyl-7-oxo-2,3-dihydro-7H-pyrido[3,2,1-ij]-1,3,4-benzoxadiazin-6-carbonsäure

100 mg 9-Fluor-3-methyl-10-[4-(4-nitrobenzyl)-1-piperazinyl]-7-oxo -2,3-dihydro-7H-pyrido[3,2,1-ij]-1,3,4-benzoxadiazin -6-carbonsäure (aus Beispiel 16) werden 2 Stunden über 10 mg 5% Pd/C in Methylenchlorid/Methanol 1:1 hydriert. Der Katalysator wird abfiltriert und das Filtrat unter vermindertem Druck zur Trockene eingedampft. Der Rückstand wird aus Aethanol umkristalisiert, und man erhält 10-[4-(4-Aminobenzyl)-1-piperazinyl]-9-fluor-3-methyl-7-oxo -2,3-dihydro-7H-pyrido[3,2,1-ij]-1,3,4-benzoxadiazin -6-carbonsäure vom Smp. 237-238 °C (Zers.); FAB-MS m/z 454 ($MH^+$).

Beispiel 20

Herstellung von 10-[3-(Aminomethyl)-1-pyrrolidinyl]-9-fluor-3-methyl-7-oxo-2,3-dihydro-7H-pyrido[3,2,1-ij]-1,3,4-benzoxadiazin-6-carbonsäure

Ein Gemisch von 40 mg 10-[3-(Acetylaminomethyl)-1-pyrrolidinyl]-9-fluor-3-methyl-7 -oxo-2,3-dihydro-7H-pyrido[3,2,1-ij]-1,3,4-benzoxadiazin -6-carbonsäure (aus Beispiel 8) und 2,5 ml 1N wässriger Natronlauge werden 3 Stunden auf 100°C erhitzt. Nach dem Erkalten auf Zimmertemperatur wird das Reaktionsgemisch mit Essigsäure neutralisiert, und der ausgefällte Niederschlag wird abfiltriert und aus Methanol umkristallisiert. Man erhält 10-[3-(Aminomethyl)-1-pyrrolidinyl]-9-fluor-3-methyl-7-oxo -2,3-dihydro-7H-pyrido[3,2,1-ij]-1,3,4-benzoxadiazin -6-carbonsäure vom Smp. 177-180°C (Zers.): FAB-MS m/z 363 ($MH^+$).

Beispiel 21

Herstellung von 6-Fluor-8-hydroxy-7-(1-imidazolyl)-1-(methylamino)-4-oxo-1,4-dihydro-3-chinolincarbonsäure

Eine Lösung von 50 mg 6,7-Difluor-8-hydroxy-1-(methylamino)-4-oxo-1,4-dihydro -3-chinolincarbonsäure (aus Bezugsbeispiel (i)) in 2 ml trockenem Dimethylformamid wird unter Rühren mit 32 mg Carbonyldiimidazol versetzt. Das Rühren wird 2 Stunden bei Zimmertemperatur und anschliessend 5 Stunden bei 80°C fortgesetzt Das Lösungsmittel wird anschliessend unter vermindertem Druck abgedampft, der Rückstand in Wasser aufgeschlämmt und der pH mit Essigsäure auf 5 eingestellt. Der ausgefällte Niederschlag wird abfiltriert una mit Methanol gewaschen. Man erhält 6-Fluor-8-hydroxy-7-(1-imidazolyl)-1-(methylamino)-4-oxo -1,4-dihydro-3-chinolincarbonsäure als schwach gelbes Pulver, FAB-MS m/z 319 ($MH^+$). $^1$H-NMR ($d_6$-DMSO) $\delta$: 2,82 (3H,s), 7,10 (1H,d,J=10,7Hz), 7,61 (1H,d), 7,75 (1H,d), 8,62 (1H,s), 8,92 (1H,s), 15,33 (1H,br,s).

Beispiel 22

Herstellung von 9-Fluor-10-(1-imidazolyl)-3-methyl-7-oxo-2,3-dihydro-7H-pyrido[3,2,1-ij]-1,3,4-benzoxadiazin-6-carbonsäure

Eine Aufschlämmung von 15 mg 6-Fluor-8-hydroxy-7-(1-imidazolyl)-1-(methylamino)-4-oxo -1,4-dihydro-3-chinolincarbonsäure (aus Beispiel 21) in einem Gemisch von 1 ml 35%igem wässrigem Formalin und 1 ml Dioxan werden 1,5 Stunden in einer Stickstoffatmosphäre auf 100-110°C erhitzt. Das Lösungsmittel wird unter vermindertem Druck abgedampft und der kristalline Rückstand mit Methanol gewaschen. Man erhält 9-Fluor-10-(1-imidazolyl)-3-methyl-7-oxo-2,3-dihydro-7H -pyrido[3,2,1-ij]-1,3,4-benzoxadiazin-6-carbonsäure als schwach rosafarbenes Pulver. Nach dem Umkristallisieren aus Dimethylformamid und Aether schmilzt die Verbindung bei >300°C; FAB-MS m/z 331 ($MH^+$).

9-Fluor-10-(1-imidazolyl)-3-methyl-7-oxo-2,3-dihydro-7H -pyrido[3,2,1-ij]-1,3,4-benzoxadiazin-6-carbonsäure ist auch aus 9,10-Difluor-3-methyl-7-oxo-2,3-dihydro-7H -pyrido[3,2,1-ij]-1,3,4-benzoxadiazin-6-carbonsäure und Imidazol in Dimethylsulfoxid in Analogie zu Beispiel 2 erhältlich.

Beispiel 23

Herstellung von Benzyl-9-fluor-10-(3-hydroxy-1-pyrrolidinyl)-3-methyl-7-oxo-2,3-dihydro-7H-pyrido[3,2,1-ij]-1,3,4-benzoxadiazin-6-carboxylat

Ein Gemisch von 10 mg 9-Fluor-10-(3-hydroxy-1-pyrrolidinyl)-3-methyl-7-oxo-2,3 -dihydro-7H-pyrido-[3,2,1-ij]-1,3,4-benzoxadiazin-6-carbonsäure (aus Beispiel 9), 8 mg wasserfreiem Kaliumcarbonat und 0,5 ml Dimethylformamid wird 1,5 Stunden bei Zimmertemperatur gerührt und anschliessend mit 10,8 mg Benzylbromid versetzt. Das Gemisch wird 3 Stunden bei Zimmertemperatur gerührt und anschliessend unter vermindertem Druck eingedampft. Der Rückstand wird in Wasser aufgeschlämmt und mit Chloroform extrahiert. Der Extrakt wird unter vermindertem Druck zur Trockene eingedampft. Der Rückstand wird mit Aether verrieben. Man erhält Benzyl-9-fluor-10-(3-hydroxy-1-pyrrolidinyl)-3-methyl-7-oxo -2,3-dihydro-7H-pyrido[3,2,1,ij]-1,3,4-benzoxadiazin -6-carboxylat vom Smp. 196-198°C (Zers.); FAB-MS m/z 440 ($MH^+$).

Beispiel 24

Herstellung von Benzyl-10-(3-chlor-1-pyrrolidinyl-9-fluor-3-methyl-7-oxo-2,3-dihydro-7H-pyrido[3,2,1-i]-1,3,4-benzoxadiazin-6-carboxylat

8 mg Benzyl-9-fluor-10-(3-hydroxy-1-pyrrolidinyl)-3-methyl-7-oxo -2,3-dihydro-7H-pyrido[3,2,1,ij]-1,3,4-benzoxadiazin -6-carboxylat (aus Beispiel 23) werden in 0,2 ml Thionylchlorid gelöst und 15 Minuten bei 60 °C gerührt. Das Reaktionsgemisch wird mit Wasser verdünnt und mit Chloroform extrahiert. Der Extrakt wird unter vermindertem Druck eingedampft. Der Rückstand wird an 2 g Kieselgel mit Chloroform chromatographiert. Man erhält Benzyl-10-(3-chlor-1-pyrrolidinyl)-9-fluor-3-methyl-7-oxo -2,3-dihydro-7H-pyrido[3,2,1-ij]-1,3,4-benzoxadiazin -6-carboxylat vom Smp. >300 °C; FAB-MS m/z ($MH^+$), 460 $(MH+2)^+$.

Beispiel 25

Herstellung von 10-(3-Chlor-1-pyrrolidinyl)-9-fluor-3-methyl-7-oxo-2,3-dihydro-7H-pyrido[3,2,1-ij]-1,3,4-benzoxadiazin-6-carbonsäure

2,5 mg Benzyl-10-(3-chlor-1-pyrrolidinyl)-9-fluor-3-methyl-7-oxo -2,3-dihydro-7H-pyrido[3,2,1-ij]-1,3,4-benzoxadiazin -6-carboxylat (aus Beispiel 24) werden über 1 mg 5% Pd/C in Chloroform hydriert. Der Katalysator wird abfiltriert und das Filtrat unter vermindertem Druck zur Trockene eingedampft, und der Rückstand wird aus Aethanol umkristallisiert. Man erhält 10-(3-Chlor-1-pyrrolidinyl)-9-fluor-3-methyl-7-oxo-2,3 -dihydro-7H-pyrido[3,2,1-ij]-1,3,4-benzoxadiazin -6-carbonsäure vom Smp. 269-272 °C (Zers.); FAB-MS m/z 368 ($MH^+$), 370 $(MH+2)^+$.

Beispiel 26

Herstellung von 9-Fluor-3-methyl-10-(4-methyl-1-piperazinyl)-7-oxo-2,3-dihydro-7H-pyrido[3,2,1-ij]-1,3,4-benzoxadiazin-6-carbonsäure über das entsprechende Fluorboran-Zwischenprodukt

(a) Ein Gemisch von 100 mg 9,10-Difluor-3-methyl-7-oxo-2,3-dihydro-7H-pyrido[3,2,1-ij] -1,3,4-benzoxadiazin-6-carbonsäure (aus Beispiel 1) und 1 ml 60% wässriger Fluorborsäure werden 12 Stunden auf 90 °C erhitzt. Das Reaktionsgemisch wird auf Zimmertemperatur abgekühlt und der Niederschlag abfiltriert, mit Methanol gewaschen und unter vermindertem Druck getrocknet. Man erhält rohes 9,10-Difluor-6-[[(difluorboryl)oxy]carbonyl]-3-methyl -2,3-dihydro-7H-pyrido[3,2,1-ij]-1,3,4-benzoxadiazin-7-on; FAB-MS m/z 331 ($MH^+$).

(b) Eine Lösung von 33 mg des obigen Boranzwischenprodukts in 1 ml Dimethylsulfoxid wird unter Rühren mit 15 µl N-Methylpiperazin und 20 µl Triäthylamin versetzt. Nach 3-stündigem Rühren bei Zimmertemperatur wird das Reaktionsgemisch lyophilisiert. Der Rückstand wird aus Methanol umkristallisiert, und man erhält 6-[[(Difluorboryl)oxy]carbonyl]-9-fluor-3-methyl-10-(4 -methyl-1-piperazinyl)-2,3-dihydro-7H-pyrido[3,2,1-ij] -1,3,4-benzoxadiazin-7-on als gelbe Kristalle vom Smp. 228-230 °C (Zers.); FAB-MS m/z 411 ($MH^+$).

(c) Eine Lösung von 5 mg des obigen Boranzwischenproduktes in 1 ml 95%igem Aethanol wird mit 3 µl Triäthylamin versetzt. Nach 4-stündigem Erhitzen unter Rückflussbedingungen wird das Reaktionsgemisch auf Zimmertemperatur abgekühlt. Der ausgefällte Niederschlag wird abfiltriert, und man erhält 9-Fluor-3-methyl-10-(4-methyl-1-piperazinyl)-7-oxo-2,3 -dihydro-7H-pyrido[3,2,1-ij]-1,3,4-benzoxadiazin -6-carbonsäure vom Smp. 268-269 °C (Zers.)..

Beispiel 27

Herstellung von 9-Fluor-3-methyl-10-(4-methyl-1-piperazinyl)-7-oxo-2,3-dihydro-7H-pyrido[3,2,1-ij]-1,3,4-benzoxadiazin-6-carbonsäure über das Acetoxyboran-Zwischenprodukt

(a) Ein Gemisch von 100 mg 9,10-Difluor-3-methyl-7-oxo-2,3-dihydro-7H-pyrido[3,2,1-ij] -1,3,4-benzoxadiazin-6-carbonsäure (aus Beispiel 1), 1 ml Essigsäureanhydrid und 100 mg Triacetoxyboran wird 15 Minuten auf 140 °C erhitzt. Das Reaktionsgemisch wird unter vermindertem Druck eingedampft. Der Rückstand wird mit Aceton verrieben und filtriert. Man erhält 6-[[(Diacetoxyboryl)oxy]carbonyl]-9,10-difluor-3-methyl-2,3 -dihydro-7H-pyrido[3,2,1-ij]-1,3,4-benzoxadiazin-7-on; FAB-MS m/z 411 ($MH^+$).

(b) Eine Lösung von 41 mg des obigen Boran-Zwischenprodukts in 1 ml Dimethylsulfoxid wird mit 15 µl N-Methylpiperazin und 20 µl Triäthylamin versetzt. Das Reaktionsgemisch wird 2 Stunden bei Zimmertemperatur gerührt und anschliessend lyophilisiert. Der Rückstand wird aus Methanol/Aether kristallisiert; man erhält 6-[[(Diacetoxyboryl)oxy]carbonyl]-9-fluor-3-methyl-10-(4 -methyl-1-piperazinyl)-2,3-dihydro-7H-pyrido[3,2,1-ij] -1,3,4-benzoxadiazin-7-on als gelbe Kristalle, Smp. 156-157 °C (Zers.); FAB-MS m/z 491 ($MH^+$).

(c) 5 mg des obigen Boran-Zwischenprodukts wird in 0,1 ml Aceton aufgeschlämmt und mit 2,5 µl konzentrierter Salzsaure versetzt. Das Reaktionsgemisch wird 30 Minuten bei Zimmertemperatur gerührt und anschliessend in einem Eisbad gekühlt. Der ausgefällte Niederschlag wird abfiltriert und in 0,1 ml 95%igem Aethanol gelöst. Die Lösung wird mit 2 µl Triäthylamin versetzt und das Gemisch 1 Stunde unter Rückflussbedingungen gehalten. Die Lösung wird auf Zimmertemperatur gekühlt und der ausgefällte Niederschlag abfiltriert. Man erhält 9-Fluor-3-methyl-10-(4-methyl-1-piperazinyl)-7-oxo-2,3 -dihydro-7H-pyrido[3,2,1-ij]-1,3,4-benzoxadiazin -6-carbonsäure vom Smp. 268-269 °C (Zers.).

Beispiel 28

Herstellung von 10-(3-Amino-1-pyrrolidinyl)-9-fluor-3-methyl-7-oxo-2,3-dihydro-7H-pyrido[3,2,1-ij]-1,3,4-benzoxadiazin-6-carbonsäure-hydrochlorid

Der pH-Wert einer Lösung von 20 mg 10-(3-Amino-1-pyrrolidinyl)-9-fluor-3-methyl-7-oxo-2,3 -dihydro-7H-pyrido[3,2,1-ij]-1,3,4-benzoxadiazin-6-carbonsäure (aus Beispiel 12) wird mit 6N Salzsäure auf 1,0 eingestellt. Die klare Lösung wird lyophilisiert und der Rückstand aus Wasser/Aethanol 1:2 kristallisiert. Man erhält 10-(3-Amino-1-pyrrolidinyl)-9-fluor-3-methyl-7-oxo-2,3 -dihydro-7H-pyrido[3,2,1-ij]-1,3,4-benzoxadiazin -6-carbonsäure-hydrochlorid vom Smp. 226-228 °C (Zers.).

Beispiel 29

Herstellung von 9-Fluor-3-methyl-10-(4-methyl-1-piperazinyl)-7-oxo-2,3-dihydro-7H-pyrido[3,2,1-ij]-1,3,4-benzoxadiazin-6-carbonsäure-hydrochlorid

9-Fluor-3-methyl-10-(4-methyl-1-piperazinyl)-7-oxo-2,3 -dihydro-7H-pyrido[3,2,1-ij]-1,3,4-benzoxadiazin -6-carbonsäure-hydrochlorid wird in Analogie zu Beispiel 28 erhalten, Smp. 264-266 °C (Zers.).

Beispiel 30

Herstellung von Natrium-9-fluor-3-methyl-10-morpholin-7-oxo-2,3-dihydro-7H-pyrido[3,2,1-ij]-1,3,4-benzoxa diazin-6-carboxylat

14 mg 9-Fluor-3-methyl-10-morpholin-7-oxo-2,3-dihydro-7H -pyrido[3,2,1-ij]-1,3,4-benzoxadiazin-6-carbonsäure (aus Beispiel 4) werden in 0,4 ml Wasser aufgeschlämmt und unter Rühren mit 40 µl 1N wässriger Natronlauge versetzt. Die klare Lösung wird lyophilisiert und der Rückstand aus Wasser/Aethanol (1:4) kristallisiert. Man erhält 9-Fluor-3-methyl-10-morpholin-7-oxo-2,3-dihydro-7H -pyrido[3,2,1-ij]-1,3,4-benzoxadiazin-6-carboxylat vom Smp. >300 °C.

Beispiel 31

Herstellung von 9-Fluor-3-(2-fluoräthyl)-10-(4-methyl-1-piperazinyl)-7-oxo-2,3-dihydro-7H-pyrido[3,2,1-ij]-1,3,4-benzoxadiazin-6-carbonsäure

9-Fluor-3-(2-fluoräthyl)-10-(4-methyl-1-piperazinyl) -7-oxo-2,3-dihydro-7H-pyrido[3,2,1-ij]-1,3,4-benzoxadiazin -6-carbonsäure wird aus Aethyl-8-benzyloxy-6,7-difluor-1-(formylamino)-4-oxo-1,4 -dihydro-3-chinolincarboxylat (aus Bezugsbeispiel (f)) mit Hilfe der in den Bezugsbeispielen (g), (h) und (i) (mit 1-Brom-2-fluoräthan anstelle von Methyljodid), Beispiel 1 und Beispiel 2 angegebenen Methoden hergestellt. Das Produkt wird nach dem umkristallisieren aus Methanol kristallin erhalten, Smp. 220-224 °C, MS m/z 394 ($M^+$).

Beispiel 32

Herstellung von Natrium-9-Fluor-3-methyl-10-(4-methyl-1-piperazinyl)-7-oxo-2,3-dihydro-7H-pyrido[3,2,1-ij]-1,3,4-benzoxadiazin-6-carboxylat

520 mg 9-Fluor-3-methyl-10-(4-methyl-1-piperazinyl)-7-oxo-2,3 -dihydro-7H-pyrido[3,2,1-ij]-1,3,4-benzoxadiazin -6-carbonsäure werden in 2,88 ml 0,5N wässriger Natriumlauge gelöst. Die klare Lösung wird unter vermindertem Druck eingedampft; das erhaltene blass-gelbe Pulver wird aus Aethanol umkristallisiert, und man erhält nach 2-tägigem Trocknen unter vermindertem Druck bei 80 °C Natrium 9-Fluor-3-methyl-10-(4-methyl-1-piperazinyl)-7-oxo-2,3 -dihydro-7H-pyrido[3,2,1-ij]-1,3,4-benzoxadiazin -6-carboxylat vom Smp. 252-254 °C (Zers.), FAB-MS m/z 385.

Die folgenden Beispiele veranschaulichen pharmazeutische Zubereitungsformen enthaltend eine erfindungsgemässe Verbindung:

Beispiel A

Es werden in üblicher Weise Gelatinekapseln mit den folgenden Bestandteilen hergestellt:

| | |
|---|---|
| 9-Fluor-3-methyl-10-(4-methyl-1-piperazinyl)-7-oxo-2,3-dihydro-7H-pyrido[3,2,1-ij]-1,3-,4-benzoxadiazin-6-carbonsäure | 200mg |
| Luviskol (wasserlösliches Polyvinylpyrrolidon) | 20mg |
| Mannit | 20mg |
| Talk | 15mg |
| Magnesiumstearat | 2mg |
| | 257mg |

Beispiel B

Es werden in üblicher Weise Tabletten mit den folgenden Bestandteilen hergestellt:

| | |
|---|---|
| 9-Fluor-3-methyl-10-(4-methyl-1-piperazinyl)-7-oxo-2,3-dihydro-7H-pyrido[3,2,1-ij]-1,3-,4-benzoxadiazin-6-carbonsäure | 200mg |
| Stärke | 44mg |
| Calciumcarboxymethylcellulose | 30mg |
| kristalline Cellulose | 40mg |
| Magnesiumstearat | 6mg |
| | 320mg |

**Patentansprüche**
**Patentansprüche für folgende Vertragsstaaten : BE, CH, DE, FR, GB, IT, LI, LU, NL, SE**

**1.** Pyrido[3,2,1-ij]-1,3,4-benzoxadiazinderivate der allgemeinen Formel

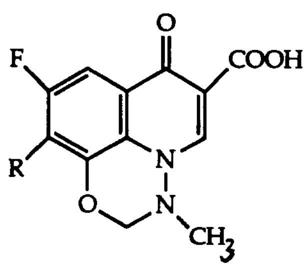

(I)

in der R

- 1-Piperazinyl, das in 4-Stellung durch Methyl, Acetyl oder 4-Aminobenzyl substituiert sein kann;
- Morpholino;
- in 3-Stellung durch Amino, Aminomethyl, (Methyl-amino)methyl, (Aethylamino)methyl, Methoxy oder Chlor substituiertes 1-Pyrrolidinyl;
- 1-Imidazolyl, das in 4-Stellung durch Methyl substituiert sein kann; oder
- in 4-Stellung durch Hydroxy oder Methoxy substituiertes 1-Piperidyl

darstellt,

sowie pharmazeutisch verträgliche Salze davon und Hydrate oder Solvate der Verbindungen der Formel I oder von deren Salzen.

**2.** Verbindungen nach Anspruch 1, dadurch gekennzeichnet, dass R die Gruppe

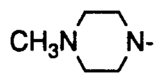

darstellt.

**3.** Verbindungen nach Anspruch 1, dadurch gekennzeichnet, dass R die Gruppe

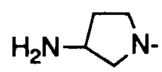

darstellt.

**4.** Verbindung nach Anspruch 1, dadurch gekennzeichnet, dass sie 9-Fluor-3-methyl-10-(4-methyl-1-piperazinyl) -7-oxo-2,3-dihydro-7H-pyrido[3,2,1-ij]-1,3,4-benzoxadiazin -6-carbonsäure oder ein pharmazeutisch verträgliches Salz davon bzw. ein Hydrat oder Solvat einer dieser Verbindungen darstellt.

**5.** Verbindung nach Anspruch 1, dadurch gekennzeichnet, dass sie 9-Fluor-3-methyl-7-oxo-10-(1-piperazinyl)-2,3-dihydro -7H-pyrido-[3,2,1-ij]-1,3,4-benzoxadiazin -6-carbonsäure oder ein pharmazeutisch verträgliches Salz davon bzw. ein Hydrat oder Solvat einer dieser Verbindungen darstellt.

**6.** Verbindung nach Anspruch 1, dadurch gekennzeichnet, dass sie 10-[3-[(Aethylamino)methyl]-1-pyrrolidinyl]-9-fluor -3-methyl-7-oxo-2,3-dihydro-7H-pyrido[3,2,1-ij] -1,3,4-benzoxadiazin-6-carbonsäure oder ein pharmazeutisch verträgliches Salz davon bzw. ein Hydrat oder Solvat einer dieser Verbindungen darstellt.

**7.** Verbindung nach Anspruch 1, dadurch gekennzeichnet, dass sie 10-(3-Amino-1-pyrrolidinyl)-9-fluor-3-methyl -7-oxo-2,3-dihydro-7H-pyrido[3,2,1-ij] -1,3,4-benzoxadiazin-6-carbonsäure oder ein pharmazeutisch verträgliches Salz davon bzw. ein Hydrat oder Solvat einer dieser Verbindungen darstellt.

**8.** Verbindung nach Anspruch 1, dadurch gekennzeichnet, dass sie 9-Fluor-3-methyl-10-[3-[(methylamino)-methyl]-1-pyrrolidinyl] -7-oxo-2,3-dihydro-7H-pyrido[3,2,1-ij]-1,3,4-benzoxadiazin -6-carbonsäure oder ein pharmazeutisch verträgliches Salz davon bzw. ein Hydrat oder Solvat einer dieser Verbindungen darstellt.

**9.** Verbindung nach Anspruch 1, dadurch gekenzeichnet, dass sie 10-[4-(4-Aminobenzyl)-1-piperazinyl]-9-fluor-3-methyl -7-oxo-2,3-dihydro-7H-pyrido[3,2,1-ij]-1,3,4-benzoxadiazin -6-carbonsäure oder ein pharmazeutisch verträgliches Salz davon bzw. ein Hydrat oder Solvat einer dieser Verbindungen darstellt.

**10.** Verbindung nach Anspruch 1, dadurch gekennzeichnet, dass sie 9- Fluor-3-methyl-10-morpholino-7-oxo-2,3-dihydro-7H-pyrido[3,2,1-ij]-1,3,4-benzoxadiazin-6-carbonsäure oder ein pharmazeutisch verträgliches Salz davon bzw. ein Hydrat oder Solvat einer dieser Verbindungen darstellt.

**11.** Verbindung nach Anspruch 1, dadurch gekennzeichnet, dass sie 9- Fluor-10-(3-methoxy-1-pyrrolidinyl)-3-methyl-7-oxo-2,3-dihydro-7H-pyrido[3,2,1-ij]-1,3,4-benzoxadiazin-6-carbonsäure oder ein pharmazeutisch vertragliches Sah davon bzw. ein Hydrat oder Solvat einer dieser Verbindungen darstellt.

**12.** Verbindung nach Anspruch 1, dadurch gekennzeichnet, dass sie 10-[3-(Aminomethyl)-1-pyrrolidinyl)-9-fluor-3-methyl-7-oxo-2,3-dihydro-7H-pyrido[3,2,1-ij]-1,3,4-benzoxadiazin-6-carbonsäure oder ein pharmazeutisch verträgliches Sah davon bzw. ein Hydrat oder Solvat einer dieser Verbindungen darstellt.

**13.** Verbindung nach Anspruch 1, dadurch gekennzeichnet, dass sie 9-Fluor-10-(1-imidazolyl)-3-methyl-7-oxo-2,3-dihydro-7H-pyrido[3,2,1-ij]-1,3,4-benzoxadiazin-6-carbonsäure oder ein pharmazeutisch verträgliches Salz davon bzw. ein Hydrat oder Solvat einer dieser Verbindungen darstellt.

**14.** Verbindung nach Anspruch 1, dadurch gekennzeichnet, dass sie 9- Fluor-3-methyl-10-(4-methyl-1-imidazolyl)-7-oxo-2,3-dihydro-7H-pyrido[3,2,1-ij]-1,3,4-benzoxadiazin-6-carbonsäure oder ein pharmazeutisch verträgliches Salz davon bzw. ein Hydrat oder Solvat einer dieser Verbindungen darstellt.

**15.** Verbindung nach Anspruch 1, dadurch gekennzeichnet, dass sie 9-Fluor-10-(4-hydroxy-1-piperidyl)-3-methyl-7-oxo-2,3-dihydro-7H-pyrido[3,2,1-ij]-1,3,4-benzoxadiazin-6-carbonsäure oder ein pharmazeutisch verträgliches Sah davon bzw. ein Hydrat oder Solvat einer dieser Verbindungen darstellt.

**16.** Verbindung nach Anspruch 1, dadurch gekennzeichnet, dass sie 10-(3-Chlor-1-pyrrolidinyl)-9-fluor-3-methyl-7-oxo-2,3-dihydro-7H-pyrido[3,2,1-ij]-1,3,4-benzoxadiazin-6-carbonsäure oder ein pharmazeutisch verträgliches Salz davon bzw. ein Hydrat oder Solvat einer dieser Verbindungen darstellt.

**17.** Verbindung nach Anspruch 1, dadurch gekennzeichnet, dass sie 10-(4-Acetyl-1-piperazinyl)-9-fluor-3-methyl-7-oxo-2,3-dihydro-7H-pyrido[3,2,1-ij]-1,3,4-benzoxadiazin-6-carbonsäure oder ein pharmazeutisch verträgliches Salz davon bzw. ein Hydrat oder Solvat einer dieser Verbindungen darstellt.

**18.** Verbindung nach Anspruch 1, dadurch gekennzeichnet, dass sie 9-Fluor-10-(4-methoxy-1-piperidyl)-3-methyl-7-oxo-2,3-dihydro-7H-pyrido[3,2,1-ij]-1,3,4-benzoxadiazin-6-carbonsäure oder ein pharmazeutisch verträgliches Salz davon bzw. ein Hydrat oder Solvat einer dieser Verbindungen darstellt.

**19.** Verbindungen der allgemeinen Formel

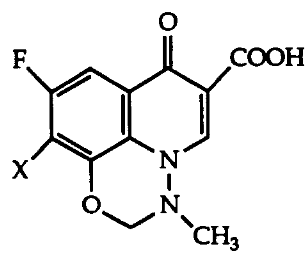

(II)

in der X ein Halogenatom darstellt.

**20.** Verbindungen der allgemeinen Formel

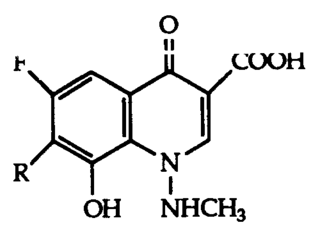

(IV)

in der R

- 1-Piperazinyl, das in 4-Stellung durch Methyl, Acetyl oder 4-Aminobenzyl substituiert sein kann;
- Morpholino;

- in 3-Stellung durch Amino, Aminomethyl, (Methyl-amino)methyl, (Aethylamino)methyl, Methoxy oder Chlor substituiertes 1-Pyrrolidinyl;
- 1-Imidazolyl, das in 4-Stellung durch Methyl substituiert sein kann; oder
- in 4-Stellung durch Hydroxy oder Methoxy substituiertes 1-Piperidyl

darstellt.

**21.** Verbindungen gemäss einem der Ansprüche 1-18 als pharmazeutische Wirkstoffe.

**22.** Verbindungen gemäss einem der Ansprüche 1-18 als pharmazeutische Wirkstoffe zur Behandlung und Prophylaxe von Infektionskrankheiten.

**23.** Verfahren zur Herstellung der Verbindungen gemäss einem der Ansprüche 1-18, dadurch gekennzeichnet, dass man

(a) eine Verbindung der allgemeinen Formel

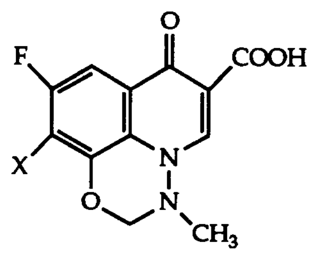

(II)

in der X ein Halogenatom darstellt; und vorhandene Amino-, Hydroxy und/oder Carboxygruppen geschützt sein können,
mit einem Amin der allgemeinen Formel

HR (III)

in der R die in Anspruch 1 gegebene Bedeutung hat,
umsetzt und notwendigenfalls eine vorhandene Schutzgruppe abspaltet, oder dass man
(b) eine Verbindung der allgemeinen Formel

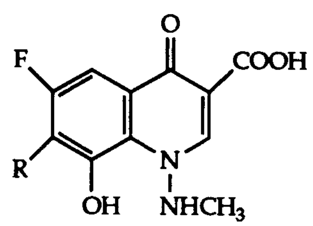

(IV)

in der R die in Anspruch 1 gegebene Bedeutung hat; und vorhandene Amino-, Hydroxy-, und/oder Carboxygruppen geschützt sein können, mit Formaldehyd oder Paraformaldehyd umsetzt und
notwendigenfalls eine vorhandene Schutzgruppe abspaltet, oder dass man
(c) zur Herstellung einer Verbindung der Formel I, worin R 4-Acetyl-1-Piperazinyl darstellt, die Verbindung der Formel I, in der R unsubstituiertes 1-Piperazinylrest darstellt, mit einem die Acetylgruppe abgebenden Mittel umsetzt, oder dass man
(d) zur Herstellung der Verbindung der Formel I, worin R 3-Methoxy-1-piperidyl darstellt, die entsprechende 3-Hydroxy-1-piperidylverbindung der Formel I methyliert, oder dass man
(e) zur Herstellung einer Verbindung der Formel I mit einer freien Aminogruppe R in einer entsprechenden Verbindung der Formel I mit einer geschützten Aminogruppe die Aminoschutzgruppe abspaltet, oder dass man
(f) zur Herstellung der Verbindung der Formel I, worin R 3-Chlor-1-pyrrolidinyldarstellt, die entsprechende 3-Hydroxy-1-pyrrolidinylverbindung mit geschützter Carboxygruppe halogeniert und die

Carboxygruppe abspaltet, oder dass man
(g) zur Herstellung der Verbindung der Formel I, worin R 4-(4-Aminobenzyl)-1-piperazinyl darstellt, die Nitrogruppe der entsprechenden 4-(4-Nitrobenzyl)-1-piperazinylverbindung reduziert, oder dass man
(h) zur Herstellung von pharmazeutisch verträglichen Salzen, Hydraten oder Solvaten einer Verbindung der Formel I bzw. von Hydraten und Solvaten besagter Salze, eine Verbindung der Formel I in ein Salz, Hydrat oder Solvat bzw. in ein Hydrat oder Solvat des besagten Salzes überführt.

**24.** Pharmazeutische Präparate, gekennzeichnet durch einen Gehalt an einer Verbindung gemäss einem der Ansprüche 1-18.

**25.** Pharmazeutische Präparate zur Behandlung und Prophylaxe von Infektionskrankheiten, gekennzeichnet durch einen Gehalt an einer Verbindung gemäss einem der Ansprüche 1-18.

**26.** Verwendung von Verbindungen gemäss einem der Ansprüche 1-18 bei der Herstellung von Arzneimitteln für die Behandlung bzw. Prophylaxe von Infektionskrankheiten.

**Patentansprüche für folgenden Vertragsstaat : GR**

**1.** Verfahren zur Herstellung von Pyrido[3,2,1-ij]-1,3,4-benzoxadiazin-derivaten der allgemeinen Formel

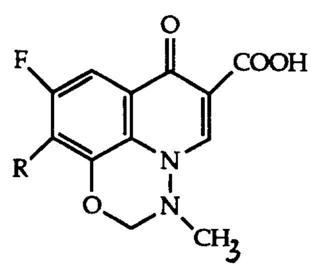

(I)

in der R
- 1-Piperazinyl, das in 4-Stellung durch Methyl, Acetyl oder 4-Aminobenzyl substituiert sein kann;
- Morpholino;
- in 3-Stellung durch Amino, Aminomethyl, (Methyl-amino)methyl, (Aethylamino)methyl, Methoxy oder Chlor substituiertes 1-Pyrrolidinyl;
- 1-Imidazolyl, das in 4-Stellung durch Methyl substituiert sein kann; oder
- in 4-Stellung durch Hydroxy oder Methoxy substituiertes 1-Piperidyl

darstellt,

sowie von pharmazeutisch verträglichen Salzen davon und von Hydraten oder Solvaten der Verbindungen der Formel I oder von deren Salzen, dadurch gekennzeichnet, dass man
(a) eine Verbindung der allgemeinen Formel

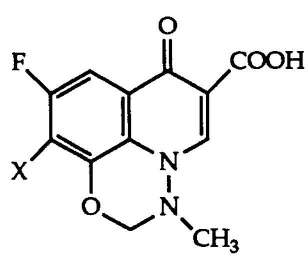

(II)

in der X ein Halogenatom darstellt; und vorhandene Amino-, Hydroxy und/oder Carboxygruppen geschützt sein können,
mit einem Amin der allgemeinen Formel

HR (III)

in der R die oben gegebene Bedeutung hat,
umsetzt und notwendigenfalls eine vorhandene Schutzgruppe abspaltet, oder dass man
(b) eine Verbindung der allgemeinen Formel

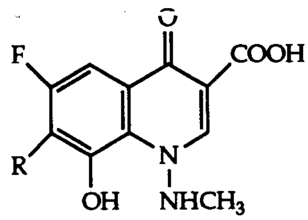

(IV)

in der R die oben gegebene Bedeutung hat; und vorhandene Amino-, Hydroxy-, und/oder Carboxygruppen geschützt sein können,
mit Formaldehyd oder Paraformaldehyd umsetzt und notwendigenfalls eine vorhandene Schutzgruppe abspaltet, oder dass man
(c) zur Herstellung einer Verbindung der Formel I, worin R 4-Acetyl-1-Piperazinyl darstellt, die Verbindung der Formel I, in der R unsubstituiertes 1-Piperazinylrest darstellt, mit einem die Acetylgruppe abgebenden Mittel umsetzt, oder dass man
(d) zur Herstellung der Verbindung der Formel I, worin R 3-Methoxy-1-piperidyl darstellt, die entsprechende 3-Hydroxy-1-piperidylverbindung der Formel I methyliert, oder dass man
(e) zur Herstellung einer Verbindung der Formel I mit einer freien Aminogruppe R in einer entsprechenden Verbindung der Formel I mit einer geschützten Aminogruppe die Aminoschutzgruppe abspaltet, oder dass man
(f) zur Herstellung der Verbindung der Formel I, worin R 3-Chlor-1-pyrrolidinyldarstellt, die entsprechende 3-Hydroxy-1-pyrrolidinylverbindung mit geschützter Carboxygruppe halogeniert und die Carboxygruppe abspaltet, oder dass man
(g) zur Herstellung der Verbindung der Formel I, worin R 4-(4-Aminobenzyl)-1-piperazinyl darstellt, die Nitrogruppe der entsprechenden 4-(4-Nitrobenzyl)-1-piperazinylverbindung reduziert, oder dass man
(h) zur Herstellung von pharmazeutisch verträglichen Salzen, Hydraten oder Solvaten einer Verbindung der Formel I bzw. von Hydraten und Solvaten besagter Salze, eine Verbindung der Formel I in ein Salz, Hydrat oder Solvat bzw. in ein Hydrat oder Solvat des besagten Salzes überführt.

**2.** Verführen zur Herstellung von Verbindungen nach Anspruch 1, worin R die Gruppe

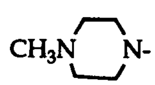

darstellt, dadurch gekennzeichnet, dass man entsprechend substituierte Ausgangsverbindungen einsetzt.

**3.** Verführen zur Herstellung von Verbindungen nach Anspruch 1, worin R die Gruppe

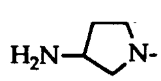

darstellt, dadurch gekennzeichnet, dass man entsprechend substituierte Ausgangsverbindungen einsetzt.

**4.** Verfahren zur Herstellung einer Verbindung nach Anspruch 1, nämlich von 9-Fluor-3-methyl-10-(4-methyl-1-piperazinyl) -7-oxo-2,3-dihydro-7H-pyrido[3,2,1-ij]-1,3,4-benzoxadiazin -6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

5. Verfahren zur Herstellung einer Verbindung nach Anspruch 1, nämlich von 9-Fluor-3-methyl-7-oxo-10-(1-piperazinyl)-2,3-dihydro -7H-pyrido-[3,2,1-ij]-1,3,4-benzoxadiazin-6-carbonsäure oder eines pharmazeutisch verträglichen Salzes davon bsw. eines Hydrats oder Solvats einer dieser Verbindungen, dadurch gekennzeichnet, dass man entsprechend substituierte Ausgangsverbindungen einsetzt.

6. Vorfahren zur Herstellung einer Verbindung nach Anspruch 1, nämlich von 10-[3-[(Aethylamino)methyl]-1-pyrrolidinyl]-9-fluor -3-methyl-7-oxo-2,3-dihydro-7H-pyrido-[3,2,1-ij] -1,3,4-benzoxadiazin-6-carbonsäure oder eines pharmazeutisch vertraglichen Salzes davon bzw. eines Hydrats oder Solvats einer dieser Verbindungen, dadurch gekennzeichnet, dass man entsprechend substituierte Ausgangsverbindungen einsetzt.

7. Verfahren zur Herstellung einer Verbindung nach Anspruch 1, nämlich von 10-(3-Amino-1-pyrrolidinyl)-9-fluor-3-methyl -7-oxo-2,3-dihydro-7H-pyrido[3,2,1-ij] -1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

8. Verfahren zur Herstellung einer Verbindung nach Anspruch 1, nämlich von 9-Fluor-3-methyl-10-[3-[-(methylamino)methyl]-1-pyrrolidinyl] -7-oxo-2,3-dihydro-7H-pyrido[3,2,1-ij]-1,3,4-benzoxadiazin -6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

9. Verfahren zur Herstellung einer Verbindung nach Anspruch 1, nämlich von 10-[4-(4-Aminobenzyl)-1-piperazinyl]-9-fluor-3-methyl -7-oxo-2,3-dihydro-7H-pyrido[3,2,1-ij]-1,3,4-benzoxadiazin -6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

10. Verfahren zur Herstellung am Verbindung nach Anspruch 1, nämlich von 9- Fluor-3-methyl-10-morpholino-7-oxo-2,3-dihydro-7H-pyrido[3,2,1-ij]-1,3,4-benzoxadiazin-6-carbonsäure oder eind pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

11. Verfahren zur Herstellung einer Verbindung nach Anspruch 1, nämlich von 9- Fluor-10-(3-methoxy-1-pyrrolidinyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

12. Verfahren zur Herstellung einer Verbindung nach Anspruch 1, nämlich von 10-[3-(Aminomethyl)-1-pyrrolidinyl)-9-fluor-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

13. Verfahren zur Herstellung einer Verbindung nach Anspruch 1, nämlich von 9-Fluor-10-(1-imidazolyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

14. Verfahren zur Herstellung einer Verbindung nach Anspruch 1, nämlich von 9- Fluor-3-methyl-10-(4-methyl-1-imidazolyl)-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

**15.** Verfahren zur Herstellung einer Verbindung nach Anspruch 1, nämlich von 9-Fluor-10-(4-hydroxy-1-piperidyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

**16.** Verfahren zur Herstellung einer Verbindung nach Anspruch 1, nämlich von 10-(3-Chlor-1-pyrrolidinyl)-9-fluor-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt..

**17.** Verfahren zur Herstellung einer Verbindung nach Anspruch 1, nämlich von 10-(4-Acetyl-1-piperazinyl)-9-fluor-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

**18.** Verfahren zur Herstellung einer Verbindung nach Anspruch 1, nämlich von 9-Fluor-10-(4-methoxy-1-piperidyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

**19.** Verfahren zur Herstellung von pharmazeutischen Präparaten, dadurch gekennzeichnet, dass man eine in Anspruch 1 definierte Verbindung der Formel I oder ein pharmazeutisch verträgliches Salz dieser Verbindung oder ein Hydrat oder Solvat einer Verbindung der Formel I bzw. eines Salzes davon als wirksamen Bestandteil mit zur therapeutischen Verabreichung geeigneten, nicht-toxischen inerten, an sich in solchen Präparaten üblichen festen und flüssigen Trägern und/oder Excipientien vermischt.

**20.** Verwendung von Verbindungen gemäss einem der Ansprüche 1-18 bei der Herstellung von Arzneimitteln für die Behandlung bzw. Prophylaxe von Infektionskrankheiten.

**21.** Verbindungen der allgemeinen Formel

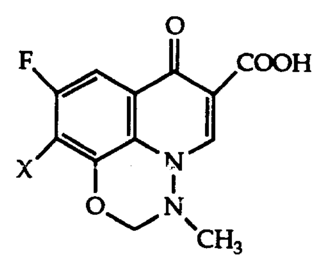

(II)

in der X ein Halogenatom darstellt.

**22.** Verbindungen der allgemeinen Formel

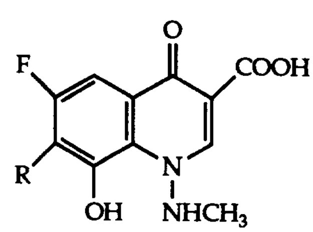

(IV)

in der R
- 1-Piperazinyl, das in 4-Stellung durch Methyl, Acetyl oder 4-Aminobenzyl substituiert sein kann;
- Morpholino;

- in 3-Stellung durch Amino, Aminomethyl, (Methyl-amino)methyl, (Aethylamino)methyl, Methoxy oder Chlor substituiertes 1-Pyrrolidinyl;
- 1-Imidazolyl, das in 4-Stellung durch Methyl substituiert sein kann; oder
- in 4-Stellung durch Hydroxy oder Methoxy substituiertes 1-Piperidyl

darstellt.

**Patentansprüche für folgende Vertragsstaaten : AT, ES**

**1.** Verfahren zur Herstellung von Pyrido[3,2,1-ij]-1,3,4-benzoxadiazin-derivaten der allgemeinen Formel

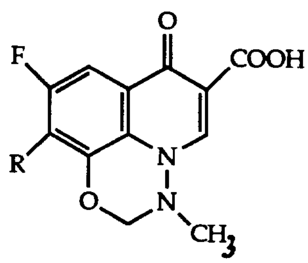

(I)

in der R
- 1-Piperazinyl, das in 4-Stellung durch Methyl, Acetyl oder 4-Aminobenzyl substituiert sein kann;
- Morpholino;
- in 3-Stellung durch Amino, Aminomethyl, (Methyl-amino)methyl, (Aethylamino)methyl, Methoxy oder Chlor substituiertes 1-Pyrrolidinyl;
- 1-Imidazolyl, das in 4-Stellung durch Methyl substituiert sein kann; oder
- in 4-Stellung durch Hydroxy oder Methoxy substituiertes 1-Piperidyl

darstellt,

sowie von pharmazeutisch verträglichen Salzen davon und von Hydraten oder Solvaten der Verbindungen der Formel I oder von deren Salzen, dadurch gekennzeichnet, dass man

(a) eine Verbindung der allgemeinen Formel

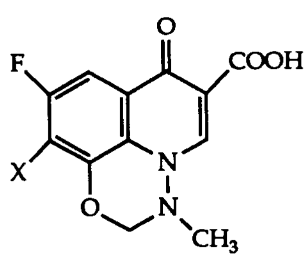

(II)

in der X ein Halogenatom darstellt; und vorhandene Amino-, Hydroxy und/oder Carboxygruppen geschützt sein können,

mit einem Amin der allgemeinen Formel

HR (III)

in der R die oben gegebene Bedeutung hat,

umsetzt und notwendigenfalls eine vorhandene Schutzgruppe abspaltet, oder dass man

(b) eine Verbindung der allgemeinen Formel

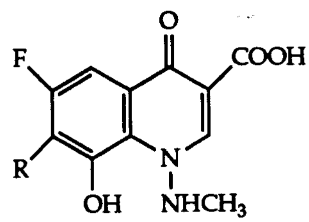

(IV)

in der R die oben gegebene Bedeutung hat; und vorhandene Amino-, Hydroxy-, und/oder Carboxygruppen geschützt sein können,
mit Formaldehyd oder Paraformaldehyd umsetzt und notwendigenfalls eine vorhandene Schutzgruppe abspaltet, oder dass man
(c) zur Herstellung einer Verbindung der Formel I, worin R 4-Acetyl-1-Piperazinyl darstellt, die Verbindung der Formel I, in der R unsubstituiertes 1-Piperazinylrest darstellt, mit einem die Acetylgruppe abgebenden Mittel umsetzt, oder dass man
(d) zur Herstellung der Verbindung der Formel I, worin R 3-Methoxy-1-piperidyl darstellt, die entsprechende 3-Hydroxy-1-piperidylverbindung der Formel I methyliert, oder dass man
(e) zur Herstellung einer Verbindung der Formel I mit einer freien Aminogruppe R in einer entsprechenden Verbindung der Formel I mit einer geschützten Aminogruppe die Aminoschutzgruppe abspaltet, oder dass man
(f) zur Herstellung der Verbindung der Formel I, worin R 3-Chlor-1-pyrrolidinyldarstellt, die entsprechende 3-Hydroxy-1-pyrrolidinylverbindung mit geschützter Carboxygruppe halogeniert und die Carboxygruppe abspaltet, oder dass man
(g) zur Herstellung der Verbindung der Formel I, worin R 4-(4-Aminobenzyl)-1-piperazinyl darstellt, die Nitrogruppe der entsprechenden 4-(4-Nitrobenzyl)-1-piperazinylverbindung reduziert, oder dass man
(h) zur Herstellung von pharmazeutisch verträglichen Salzen, Hydraten oder Solvaten einer Verbindung der Formel I bzw. von Hydraten und Solvaten besagter Salze, eine Verbindung der Formel I in ein Salz, Hydrat oder Solvat bzw. in ein Hydrat oder Solvat des besagten Salzes überführt.

**2.** Verfahren zur Herstellung von Verbindungen nach Anspruch 1, worin R die Gruppe

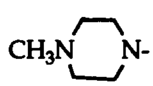

darstellt, dadurch gekennzeichnet, dass man entsprechend substituierte Ausgangsverbindungen einsetzt.

**3.** Verfahren zur Herstellung von Verbindungen nach Anspruch 1, worin R die Gruppe

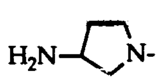

darstellt, dadurch gekennzeichnet, dass man entsprechend substituierte Ausgangsverbindungen einsetzt.

**4.** Verfahren zur Herstellung einer Verbindung nach Anspruch 1, nämlich von 9-Fluor-3-methyl-10-(4-methyl-1-piperazinyl) -7-oxo-2,3-dihydro-7H-pyrido[3,2,1-ij]-1,3,4-benzoxadiazin -6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

5. Verfahren zur Herstellung einer Verbindung nach Anspruch 1, nämlich von 9-Fluor-3-methyl-7-oxo-10-(1-piperazinyl)-2,3-dihydro -7H-pyrido-[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

6. Verfahren zur Herstellung einer Verbindung nach Anspruch 1, nämlich von 10-[3-[(Aethylamino)methyl]-1-pyrrolidinyl]-9-fluor -3-methyl-7-oxo-2,3-dihydro-7H-pyrido-[3,2,1-ij] -1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

7. Verfahren zur Herstellung einer Verbindung nach Anspruch 1, nämlich von 10-(3-Amino-1-pyrrolidinyl)-9-fluor-3-methyl -7-oxo-2,3-dihydro-7H-pyrido[3,2,1-ij] -1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

8. Verfahren zur Herstellung einer Verbindung nach Anspruch 1, nämlich von 9-Fluor-3-methyl-10-[3-[-(methylamino)methyl]-1-pyrrolidinyl] -7-oxo-2,3-dihydro-7H-pyrido[3,2,1-ij]-1,3,4-benzoxadiazin -6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

9. Verfahren zur Herstellung einer Verbindung nach Anspruch 1, nämlich von 10-[4-(4-Aminobenzyl)-1-piperazinyl]-9-fluor-3-methyl -7-oxo-2,3-dihydro-7H-pyrido[3,2,1-ij]-1,3,4-benzoxadiazin -6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

10. Verfahren zur Herstellung einer Verbindung nach Anspruch 1, nämlich von 9- Fluor-3-methyl-10-morpholino-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

11. Verfahren zur Herstellung einer Verbindung nach Anspruch 1, nämlich von 9- Fluor-10-(3-methoxy-1-pyrrolidinyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

12. Verfahren zur Herstellung einer Verbindung nach Anspruch 1, nämlich von 10-[3-(Aminomethyl)-1-pyrrolidinyl)-9-fluor-5-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

13. Verfahren zur Herstellung einer Verbindung nach Anspruch 1, nämlich von 9-Fluor-10-(1-imidazolyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

14. Verfahren zur Herstellung einer Verbindung nach Anspruch 1, nämlich von 9- Fluor-3-methyl-10-(4-methyl-1-imidazolyl)-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

15. Verfahren zur Herstellung einer Verbindung nach Anspruch 1, nämlich von 9-Fluor-10-(4-hydroxy-1-piperidyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

**16.** Verfahren zur Herstellung einer Verbindung nach Anspruch 1, nämlich von 10-(3-Chlor-1-pyrrolidinyl)-9-fluor-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt..

**17.** Verfahren zur Herstellung einer Verbindung nach Anspruch 1, nämlich von 10-(4-Acetyl-1-piperazinyl)-9-fluor-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

**18.** Verfahren zur Herstellung einer Verbindung nach Anspruch 1, nämlich von 9-Fluor-10-(4-methoxy-1-piperidyl)-3-methyl-7-oxo-2,3-dihydro-7H-pyrido[3,2,1-ij]-1,3,4-benzoxadiazin-6-carbonsäure oder eines pharmazeutisch verträglichen Salzes davon bzw. eines Hydrats oder Solvats einer dieser Verbindungen, dadurch gekennzeichnet, dass man entsprechend substituierte Ausgangsverbindungen einsetzt.

**19.** Verfahren zur Herstellung von pharmazeutischen Präparaten, dadurch gekennzeichnet, dass man eine in Anspruch 1 definierte Verbindung der Formel I oder ein pharmazeutisch verträgliches Salz dieser Verbindung oder ein Hydrat oder Solvat einer Verbindung der Formel I bzw. eines Salzes davon als wirksamen Bestandteil mit zur therapeutischen Verabreichung geeigneten, nicht-toxischen inerten, an sich in solchen Präparaten üblichen festen und flüssigen Trägern und/oder Excipientien vermischt.

**20.** Verwendung von Verbindungen gemäss einem der Ansprüche 1-18 bei der Herstellung von Arzneimitteln für die Behandlung bzw. Prophylaxe von Infektionskrankheiten.

**Claims**

**Claims for the following Contracting States : BE, CH, DE, FR, GB, IT, LI, LU, NL, SE**

**1.** Pyrido [3,2,1-ij]-1,3,4-benzoxadiazine derivatives of the general formula

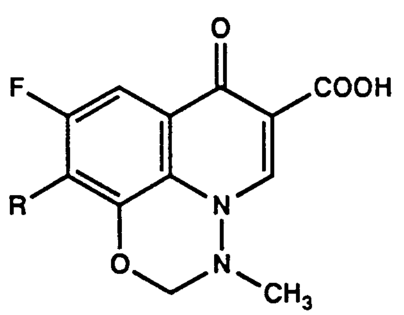

I

in which R represents
1-piperazinyl which can be substituted in the 4-position by methyl, acetyl or 4-aminobenzyl;
morpholino;
1-pyrrolidino substituted in the 3-position by amino, aminomethyl, (methyl-amino)methyl, (ethyl-amino)methyl, methoxy or chlorine;
1-imidazolyl which can be substituted in the 4-position by methyl; or
1-piperidyl substituted in the 4-position by hydroxy or methoxy,
as well as pharmaceutically acceptable salts thereof and hydrates or solvates of the compounds of formula I or of their salts.

2. Compounds according to claim 1, characterized in that R represents the group

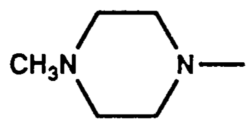

.

3. Compounds according to claim 1, characterized in that R represents the group

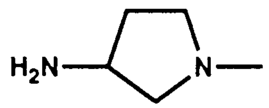

.

4. A compound according to claim 1, characterized in that it is 9-fluoro-3-methyl-10-(4-methyl-1-piperazinyl)-7-oxo-2,3-dihydro-7H-pyrido[3,2,1-ij]-1,3,4-benzoxadiazine-6-carboxylic acid or a pharmaceutically acceptable salt thereof or a hydrate of solvate of one of these compounds.

5. A compound according to claim 1, characterized in that it is 9-fluoro-3-methyl-7-oxo-10-(1-piperazinyl)-2,3-dihydro-7H-pyrido[3,2,1-ij]-1,3,4-benzoxadiazine-6-carboxylic acid or a pharmaceutically acceptable salt thereof or a hydrate of solvate of one of these compounds.

6. A compound according to claim 1, characterized in that it is 10-[3-[(ethylamino)methyl]-1-pyrrolidinyl]-9-fluoro-3-methyl-7-oxo-2,3-dihydro-7H-pyrido[3,2,1-ij]-1,3,4-benzoxadiazine-6-carboxylic acid or a pharmaceutically acceptable salt thereof or a hydrate of solvate of one of these compounds.

7. A compound according to claim 1, characterized in that it is 10-(3-amino-1-pyrrolidinyl)-9-fluoro-3-methyl-7-oxo-2,3-dihydro-7H-pyrido[3,2,1-ij]-1,3,4-benzoxadiazine-6-carboxylic acid or a pharmaceutically acceptable salt thereof or a hydrate of solvate of one of these compounds.

8. A compound according to claim 1, characterized in that it is 9-fluoro-3-methyl-10-[3-[(methylamino)-methyl]-1-pyrrolidinyl]-7-oxo-2,3-dihydro-7H-pyrido[3,2,1-ij]-1,3,4-benzoxadiazine-6-carboxylic acid or a pharmaceutically acceptable salt thereof or a hydrate of solvate of one of these compounds.

9. A compound according to claim 1, characterized in that it is 10-[4-(4-aminobenzyl)-1-piperazinyl]-9-fluoro-3-methyl-7-oxo-2,3-dihydro-7H-pyrido[3,2,1-ij]-1,3,4-benzoxadiazine-6-carboxylic acid or a pharmaceutically acceptable salt thereof or a hydrate of solvate of one of these compounds.

10. A compound according to claim 1, characterized in that it is 9-fluoro-3-methyl-10-morpholino-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

11. A compound according to claim 1, characterized in that it is 9-fluoro-10-(3-methoxy-1-pyrrolidinyl)-3-methyl-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

12. A compound according to claim 1, characterized in that it is 10-[3-(aminomethyl)-1-pyrrolidinyl)-9-fluoro-3-methyl-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

13. A compound according to claim 1, characterized in that it is 9-fluoro-10-(1-imidazolyl)-3-methyl-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

**14.** A compound according to claim 1, characterized in that it is 9-fluoro-3-methyl-10-(4-methyl-1-imidazolyl)-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

**15.** A compound according to claim 1, characterized in that it is 9-fluoro-10-(4-hydroxy-1-piperidyl)-3-methyl-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

**16.** A compound according to claim 1, characterized in that it is 10-(3-chloro-1-pyrrolidinyl)-9-fluoro-3-methyl-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

**17.** A compound according to claim 1, characterized in that it is 10-(4-acetyl-1-piperazinyl)-9-fluoro-3-methyl-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

**18.** A compound according to claim 1, characterized in that it is 9-fluoro-10-(4-methoxy-1-piperidyl)-3-methyl-7-oxo-2,3-dihydro-7H-pyrido[3,2,1-ij]-1,3,4-benzoxadiazine-6-carboxylic acid or a pharmaceutically acceptable salt thereof or a hydrate or solvate of one of these compounds.

**19.** Compounds of the general formula

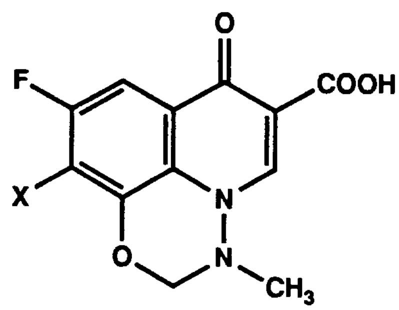

II

in which X represents a halogen atom.

**20.** Compounds of the general formula

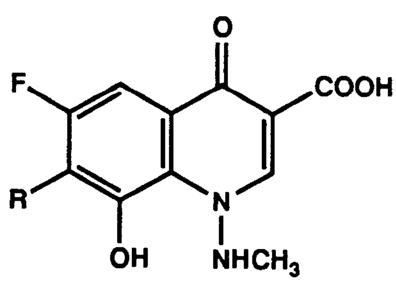

IV

in which R represents
1-piperazinyl which can be substituted in the 4-position by methyl, acetyl or 4-aminobenzyl;
morpholino;
1-pyrrolidino substituted in the 3-position by amino, aminomethyl, (methyl-amino)methyl, (ethyl-amino)methyl, methoxy or chlorine;
1-imidazolyl which can be substituted in the 4-position by methyl; or
1-piperidyl substituted in the 4-position by hydroxy or methoxy.

**21.** Compounds according to any one of claims 1-18 as pharmaceutically active substances.

**22.** Compounds according to any one of claims 1-18 as pharmaceutically active substances for the treatment and prophylaxis of infectious diseases.

**23.** A process for the manufacture of the compounds in accordance with any one of claims 1-18, characterized by

(a) reacting a compound of the general formula

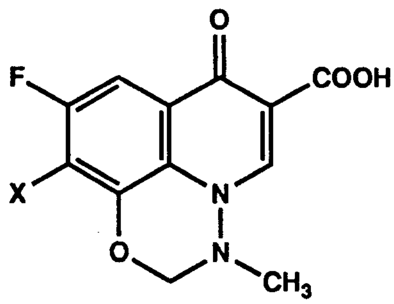

II

in which X represents a halogen atom; and amino, hydroxy and/or carboxy groups present may be protected,
with an amine of the general formula

HR III

in which R has the significance given in claim 1,
and, if necessary, cleaving off a protecting group present, or
(b) reacting a compound of the general formula

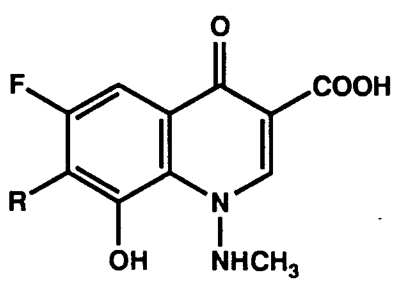

IV

in which R has the significance given in claim 1; and amino, hydroxy and/or carboxy groups present may be protected,
with formaldehyde or paraformaldehyde and, if necessary, cleaving off a protecting group present, or
(c) for the manufacture of a compound of formula I in which R represents 4-acetyl-1-piperazinyl, reacting the compound of formula I in which R represents the unsubstituted 1-piperazinyl residue with an agent yielding the acetyl group, or
(d) for the manufacture of a compound of formula I in which R represents 3-methoxy-1-piperidyl, methylating the corresponding 3-hydroxy-1-piperidyl compound of formula I, or
(e) for the manufacture of a compound of formula I having a free amino group R, cleaving off the amino protecting group in a corresponding compound of formula I having a protected amino group, or
(f) for the manufacture of a compound of formula I in which R represents 3-chloro-1-pyrrolidinyl, halogenating the corresponding 3-hydroxy-1-pyrrolidinyl compound having a protected carboxy group and cleaving off the carboxy group, or
(g) for the manufacture of a compound of formula I in which R represents 4-(4-aminobenzyl)-1-piperazinyl, reducing the nitro group of the corresponding 4-(4-nitrobenzyl)-1-piperazinyl compound, or

(h) for the manufacture of pharmaceutically acceptable salts, hydrates or solvates of a compound of formula I or of hydrates or solvates of said salts, converting a compound of formula I into a salt, hydrate or solvate or into a hydrate or solvate of said salt.

**24.** A pharmaceutical preparation, characterized in that it contains a compound in accordance with any one of claims 1-18.

**25.** A pharmaceutical preparation for the treatment and prophylaxis of infectious diseases, characterized in that it contains a compound in accordance with any one of claims 1-18.

**26.** The use of the compounds in accordance with any one of claims 1-18 for the manufacture of medicaments for the treatment and prophylaxis of infectious diseases.

**Claims for the following Contracting State : GR**

**1.** A process for the manufacture of pyrido[3,2,1-ij]-1,3,4-benzoxadiazine derivatives of the general formula

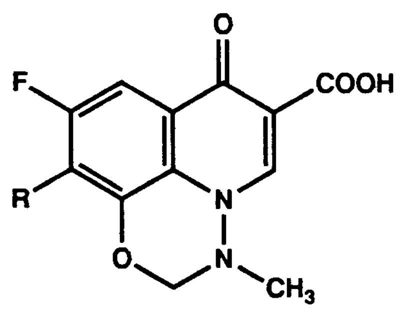

I

in which R represents
1-piperazinyl which can be substituted in the 4-position by methyl, acetyl or 4-aminobenzyl;
morpholino;
1-pyrrolidino substituted in the 3-position by amino, aminomethyl, (methyl-amino)methyl, (ethyl-amino)methyl, methoxy or chlorine;
1-imidazolyl which can be substituted in the 4-position by methyl; or
1-piperidyl substituted in the 4-position by hydroxy or methoxy,
as well as of pharmaceutically acceptable salts thereof and hydrates or solvates of the compounds of formula I or of their salts, characterized by
(a) reacting a compound of the general formula

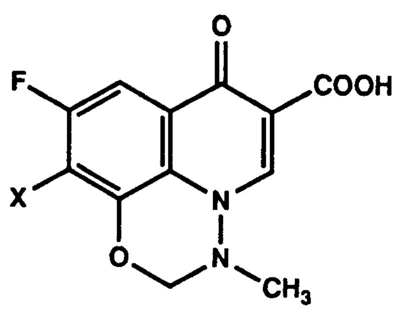

II

in which X represents a halogen atom; and amino, hydroxy and/or carboxy groups present may be protected
with an amine of the general formula

HR III

in which R has the significance given above,
and, if necessary, cleaving off a protecting group present, or
(b) reacting a compound of the general formula

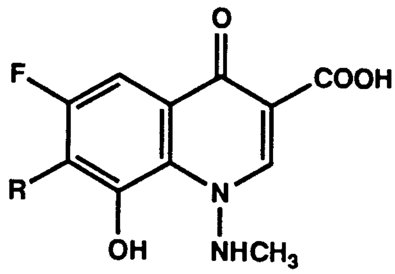

IV

in which R has the significance given above; and amino, hydroxy and/or carboxy groups present may be protected,
with formaldehyde or paraformaldehyde and, if necessary, cleaving off a protecting group present, or
(c) for the manufacture of a compound of formula I in which R represents 4-acetyl-1-piperazinyl, reacting the compound of formula I in which R represents the unsubstituted 1-piperazinyl residue with an agent yielding the acetyl group, or
(d) for the manufacture of a compound of formula I in which R represents 3-methoxy-1-piperidyl, methylating the corresponding 3-hydroxy-1-piperidyl compound of formula I, or
(e) for the manufacture of a compound of formula I having a free amino group R, cleaving off the amino protecting group in a corresponding compound of formula I having a protected amino group, or
(f) for the manufacture of a compound of formula I in which R represents 3-chloro-1-pyrrolidinyl, halogenating the corresponding 3-hydroxy-1-pyrrolidinyl compound having a protected carboxy group and cleaving off the carboxy group, or
(g) for the manufacture of a compound of formula I in which R represents 4-(4-aminobenzyl)-1-piperazinyl, reducing the nitro group of the corresponding 4-(4-nitrobenzyl)-1-piperazinyl compound, or
(h) for the manufacture of pharmaceutically acceptable salts, hydrates or solvates of a compound of formula I or of hydrates or solvates of said salts, converting a compound of formula I into a salt, hydrate or solvate or into a hydrate or solvate of said salt.

**2.** A process for the manufacture of compounds according to claim 1 in which R represents the group

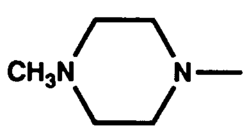

,

characterized in that corresponding starting compounds are used.

**3.** A process for the manufacture of compounds according to claim 1 in which R represents the group

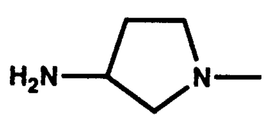

,

characterized in that corresponding starting compounds are used.

**4.** A process for the manufacture of a compound according to claim 1 which is 9-fluoro-3-methyl-10-(4-methyl-1-piperazinyl)-7-oxo-2,3-dihydro-7H-pyrido[3,2,1-ij]-1,3,4-benzoxadiazine-6-carboxylic acid or of

a pharmaceutically acceptable salt thereof or of a hydrate or solvate of one of these compounds, characterized in that correspondingly substituted starting compounds are used.

5. A process for the manufacture of a compound according to claim 1 which is 9-fluoro-3-methyl-7-oxo-10-(1-piperazinyl)-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

6. A process for the manufacture of a compound according to claim 1 which is 10-[3-[(ethylamino)methyl]-1-pyrrolidinyl]-9-fluoro-3-methyl-7-oxo-2,3-dhydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

7. A process for the manufacture of a compound according to claim 1 which is 10-(3-amino-1-pyrrolidinyl)-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

8. A process for the manufacture of a compound according to claim 1 which is 9-fluoro-3-methyl-10-[3-[-(methylamino)methyl]-1-pyrrolidinyl]-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

9. A process for the manufacture of a compound according to claim 1 which is 10-[4-(4-aminobenzyl)-1-piperazinyl]-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

10. A process for the manufacture of a compound according to claim 1 which is 9-fluoro-3-methyl-10-morpholino-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

11. A process for the manufacture of a compound according to claim 1 which is 9-fluoro-10-(3-methoxy-1-pyrrolidinyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

12. A process for the manufacture of a compound according to claim 1 which is 10-[3-(aminomethyl)-1-pyrrolidinyl)-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

13. A process for the manufacture of a compound according to claim 1 which is 9-fluoro-10-(1-imidazolyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

14. A process for the manufacture of a compound according to claim 1 which is 9-fluoro-3-methyl-10-(4-methyl-1-imidazolyl)-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

15. A process for the manufacture of a compound according to claim 1 which is 9-fluoro-10-(4-hydroxy-1-piperidyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**16.** A process for the manufacture of a compound according to claim 1 which is 10-(3-chloro-1-pyrrolidinyl)-9-fluor-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**17.** A process for the manufacture of a compound according to claim 1 which is 10-(4-acetyl-1-piperazinyl)-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or, solvate of one of these compounds, characterized in that corresponding starting compounds are used.

**18.** A process for the manufacture of a compound according to claim 1 which is 9-fluoro-10-(4-methoxy-1-piperidyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**19.** A process for the manufacture of pharmaceutical, preparations, characterized by mixing a compound of formula I defined in claim 1 or a pharmaceutically acceptable salt of this compound or a hydrate or solvate of a compound of formula I or of a salt thereof as the active ingredient with non-toxic, inert, solid and liquid carriers and/or excipients which are usual in such preparations and which are suitable for therapeutic administration.

**20.** The use of compounds in accordance with any one of claims 1-18 for the manufacture of medicaments for the treatment and prophylaxis of infectious diseases.

**21.** Compounds of the general formula

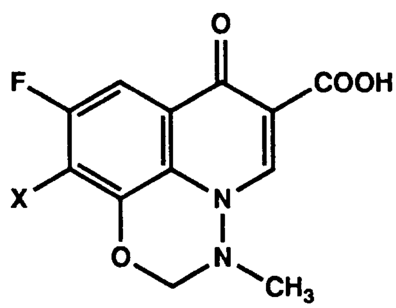

II

in which X represents a halogen atom.

**22.** Compounds of the general formula

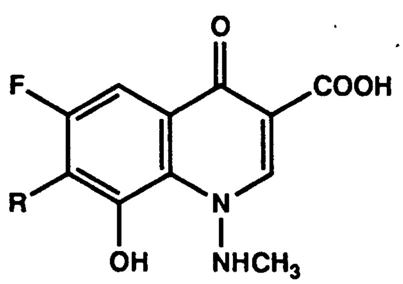

IV

in which R represents
1-piperazinyl which can be substituted in the 4-position by methyl, acetyl or 4-aminobenzyl;
morpholino;
1-pyrrolidino substituted in the 3-position by amino, aminomethyl, (methyl-amino)methyl, (ethyl-amino)methyl, methoxy or chlorine;

1-imidazolyl which can be substituted in the 4-position by methyl; or
1-piperidyl substituted in the 4-position by hydroxy or methoxy.

**Claims for the following Contracting States : AT, ES**

**1.** A process for the manufacture of pyrido[3,2,1-ij]-1,3,4-benzoxadiazine derivatives of the general formula

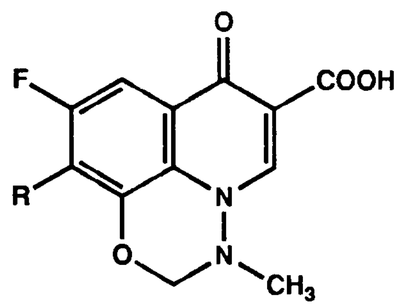

I

in which R represents
1-piperazinyl which can be substituted in the 4-position by methyl, acetyl or 4-aminobenzyl;
morpholino;
1-pyrrolidino substituted in the 3-position by amino, aminomethyl, (methyl-amino)methyl, (ethyl-amino)methyl, methoxy or chlorine;
1-imidazolyl which can be substituted in the 4-position by methyl; or
1-piperidyl substituted in the 4-position by hydroxy or methoxy,
as well as pharmaceutically acceptable salts thereof and hydrates or solvates of the compounds of formula I or of their salts, characterized by
(a) reacting a compound of the general formula

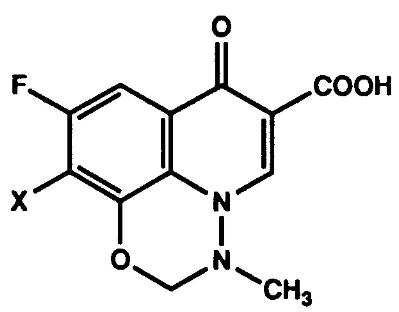

II

in which X represents a halogen atom; and amino, hydroxy and/or carboxy groups present may be protected,
with an amine of the general formula

HR III

in which R has the significance given above,
and, if necessary, cleaving off a protecting group present, or

(b) reacting a compound of the general formula

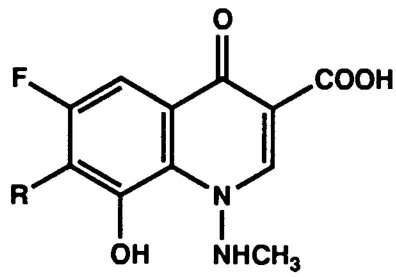

IV

in which R has the significance given above;and amino, hydroxy and/or carboxy groups present may be protected,

with formaldehyde or paraformaldehyde and, if necessary, cleaving off a protecting group present, or

(c) for the manufacture of a compound of formula I in which R represents 4-acetyl-1-piperazinyl, reacting the compound of formula I in which R represents the unsubstituted 1-piperazinyl residue with an agent yielding the acetyl group, or

(d) for the manufacture of a compound of formula I in which R represents 3-methoxy-1-piperidyl, methylating the corresponding 3-hydroxy-1-piperidyl compound of formula I, or

(e) for the manufacture of a compound of formula I having a free amino group R, cleaving off the amino protecting group in a corresponding compound of formula I having a protected amino group, or

(f) for the manufacture of a compound of formula I in which R represents 3-chloro-1-pyrrolidinyl, halogenating the corresponding 3-hydroxy-1-pyrrolidinyl compound having a protected carboxy group and cleaving off the carboxy group, or

(g) for the manufacture of a compound of formula I in which R represents 4-(4-aminobenzyl)-1-piperazinyl, reducing the nitro group of the corresponding 4-(4-nitrobenzyl)-1-piperazinyl compound, or

(h) for the manufacture of pharmaceutically acceptable salts, hydrates or solvates of a compound of formula I or of hydrates or solvates of said salts, converting a compound of formula I into a salt, hydrate or solvate or into a hydrate or solvate of said salt.

2. A process for the manufacture of compounds according to claim 1 in which R represents the group

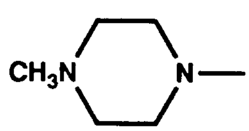

,

characterized in that corresponding starting compounds are used.

3. A process for the manufacture of compounds according to claim 1 in which R represents the group

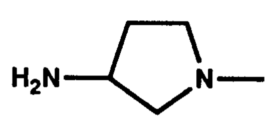

,

characterized in that corresponding starting compounds are used.

4. A process for the manufacture of a compound according to claim 1 which is 9-fluoro-3-methyl-10-(4-methyl-1-piperazinyl)-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

**5.** A process for the manufacture of a compound according to claim 1 which is 9-fluoro-3-methyl-7-oxo-10-(1-piperazinyl)-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

**6.** A process for the manufacture of a compound according to claim 1 which is 10-[3-[(ethylamino)methyl]-1-pyrrolidinyl]-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

**7.** A process for the manufacture of a compound according to claim 1 which is 10-(3-amino-1-pyrrolidinyl)-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

**8.** A process for the manufacture of a compound according to claim 1 which is 9-fluoro-3-methyl-10-[3-[-(methylamino)methyl]-1-pyrrolidinyl]-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

**9.** A process for the manufacture of a compound according to claim 1 which is 10-[4-(4-aminobenzyl)-1-piperazinyl]-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that correspondingly substituted starting compounds are used.

**10.** A process for the manufacture of a compound according to claim 1 which is 9-fluoro-3-methyl-10-morpholino-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**11.** A process for the manufacture of a compound according to claim 1 which is 9-fluoro-10-(3-methoxy-1-pyrrolidinyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**12.** A process for the manufacture of a compound according to claim 1 which is 10-[3-(aminomethyl)-1-pyrrolidinyl)-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**13.** A process for the manufacture of a compound according to claim 1 which is 9-fluoro-10-(1-imidazolyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**14.** A process for the manufacture of a compound according to claim 1 which is 9-fluoro-3-methyl-10-(4-methyl-1-imidazolyl)-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**15.** A process for the manufacture of a compound according to claim 1 which is 9-fluoro-10-(4-hydroxy-1-piperidyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**16.** A process for the manufacture of a compound according to claim 1 which is 10-(3-chloro-1-pyrrolidinyl)-9-fluoro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds,

characterized in that corresponding starting compounds are used.

**17.** A process for the manufacture of a compound according to claim 1 which is 10-(4-acetyl-1-piperazinyl)-9-fluro-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**18.** A process for the manufacture of a compound according to claim 1 which is 9-fluoro-10-(4-methoxy-1-piperidyl)-3-methyl-7-oxo-2,3-dihydro-7H-pyrido[3,2,1-ij]-1,3,4-benzoxadiazine-6-carboxylic acid or of a pharmaceutically acceptable salt thereof or of a hydrate or solvate of one of these compounds, characterized in that corresponding starting compounds are used.

**19.** A process for the manufacture of pharmaceutical, preparations, characterized by mixing a compound of formula I defined in claim 1 or a pharmaceutically acceptable salt of this compound or a hydrate or solvate of a compound of formula I or of a salt thereof as the active ingredient with non-toxic, inert, solid and liquid carriers and/or excipients which are usual in such preparations and which are suitable for therapeutic administration.

**20.** The use of compounds in accordance with any one of claims 1-18 for the manufacture of medicaments for the treatment and prophylaxis of infectious diseases.

**Revendications**

**Revendications pour les Etats contractants suivants : BE, CH, DE, FR, GB, IT, LI, LU, NL, SE**

**1.** Dérivés de pyrido[3,2,1-ij]-1,3,4-benzoxadiazine de formule générale

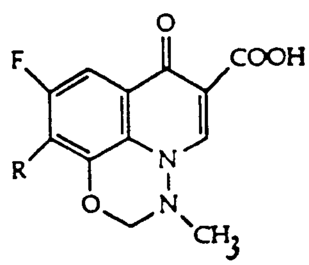

(I)

dans laquelle R représente

- un radical 1-pipérazinyle qui peut être substitué en position 4 par un groupe méthyle, acétyle ou 4-aminobenzyle;
- le radical morpholino;
- un radical 1-pyrrolidinyle substitué en position 3 par un atome de chlore ou par un groupe amino, aminométhyle, (méthylamino)méthyle, (éthylamino)méthyle ou méthoxy;
- un radical 1-imidazolyle qui peut être substitué en position 4 par le groupe méthyle; ou
- un radical 1-pipéridyle substitué en position 4 par un groupe hydroxy ou méthoxy,

ainsi que leurs sels pharmaceutiquement acceptables et les hydrates ou produits de solvatation des composés de formule I ou de leurs sels.

**2.** Composés selon la revendication 1, caractérisés en ce que R représente le groupe

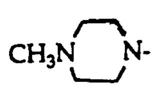

.

**3.** Composés selon la revendication 1, caractérisés en ce que R représente le groupe

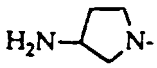

.

**4.** Composé selon la revendication 1, caractérisé en ce qu'il représente l'acide 9-fluoro-3-méthyl-10-(4-méthyl-1-pipérazinyl)-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**5.** Composé selon la revendication 1, caractérisé en ce qu'il représente l'acide 9-fluoro-3-méthyl-7-oxo-10-(1-pipérazinyl)-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composes.

**6.** Composé selon la revendication 1, caractérisé en ce qu'il représente l'acide 10-[3-[(éthylamino)méthyl]-1-pyrrolidinyl]-9-fluoro-3-méthyl-7-oxo-2,3-dihydro-7H-pyrido-[3,2,1-ij]-1,3,4-benzoxadiazine-6-carboxylique ou un sel pharmaceutiquement acceptable de celui-ci, ou un hydrate ou produit de solvatation de l'un de ces composés.

**7.** Composé selon la revendication 1, caractérisé en ce qu'il représente l'acide 10-(3-amino-1-pyrrolidinyl)-9-fluoro-3-méthyl-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composes.

**8.** Composé selon la revendication 1, caractérisé en ce qu'il représente l'acide 9-fluoro-3-méthyl-10-[3-[-(méthylamino)méthyl]-1-pyrrolidinyl]-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**9.** Composé selon la revendication 1, caractérisé en ce qu'il représente l'acide 10-[4-(4-aminobenzyl)-1-pipérazinyl]-9-fluoro-3-méthyl-7-oxo-2,3-dihydro-7H-pyrido-[3,2,1-ij]-1,3,4-benzoxadiazine-6-carboxylique ou un sel pharmaceutiquement acceptable de celui-ci, ou un hydrate ou produit de solvatation de l'un de ces composés.

**10.** Composé selon la revendication 1, caractérisé en ce qu'il représente l'acide 9-fluoro-3-méthyl-10-morpholino-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**11.** Composé selon la revendication 1, caractérisé en ce qu'il représente l'acide 9-fluoro-10-(3-méthoxy-1-pyrrolidinyl)-3-méthyl-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**12.** Composé selon la revendication 1, caractérisé en ce qu'il représente l'acide 10-[3-(aminométhyl)-1-pyrrolidinyl)-9-fluoro-3-méthyl-7-oxo-2,3-dihydro-7H-pyrido-[3,2,1-ij]-1,3,4-benzoxadiazine-6-carboxylique ou un sel pharmaceutiquement acceptable de celui-ci, ou un hydrate ou produit de solvatation de l'un de ces composés.

**13.** Composé selon la revendication 1, caractérisé en ce qu'il représente l'acide 9-fluoro-10-(1-imidazolyl)-3-méthyl-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**14.** Composé selon la revendication 1, caractérisé en ce qu'il représente l'acide 9-fluoro-3-méthyl-10-(4-méthyl-1-imidazolyl)-7-oxo-2,3-dihydro-7H-pyrido[3,2,1-ij]-benzoxadiazine-6-carboxylique ou un sel pharmaceutiquement acceptable de celui-ci, ou un hydrate ou produit de solvatation de l'un de ces composés.

**15.** Composé selon la revendication 1, caractérisé en ce qu'il représente l'acide 9-fluoro-10-(4-hydroxy-1-pipéridyl)-3-méthyl-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**16.** Composé selon la revendication 1, caractérisé en ce qu'il représente l'acide 10-(3-chloro-1-pyrrolidinyl)-9-fluoro-3-méthyl-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**17.** Composé selon la revendication 1, caractérisé en ce qu'il représente l'acide 10-(4-acétyl-1-pipérazinyl)-9-fluoro-3-méthyl-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**18.** Composé selon la revendication 1, caractérisé en ce qu'il représente l'acide 9-fluoro-10-(4-méthoxy-1-pipéridyl)-3-méthyl-7-oxo-2,3-dihydro-7H-pyrido[3,2,1-ij]-1,3,4-benzoxadiazine-6-carboxylique ou un sel pharmaceutiquement acceptable de celui-ci, ou un hydrate ou produit de solvatation de l'un de ces composés.

**19.** Composés de formule générale

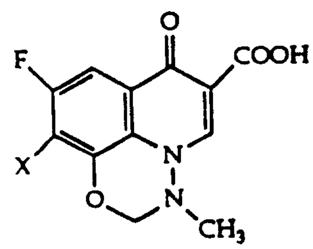

(II)

dans laquelle X représente un atome d'halogène.

**20.** Composés de formule générale

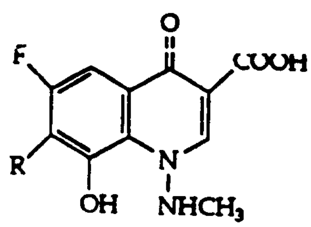

(IV)

dans laquelle R représente

- un radical 1-pipérazinyle qui peut être substitué en position 4 par un groupe méthyle, acétyle ou 4-aminobenzyle;
- le radical morpholino;
- un radical 1-pyrrolidinyle substitué en position 3 par un atome de chlore ou par un groupe amino, aminométhyle, (méthylamino)méthyle, (éthylamino)méthyle ou méthoxy;
- un radical 1-imidazolyle qui peut être substitué en position 4 par le groupe méthyle; ou
- un radical 1-pipéridyle substitué en position 4 par un groupe hydroxy ou méthoxy.

**21.** Composés selon l'une des revendications 1 à 18, en tant que substances actives pharmaceutiques.

**22.** Composés selon l'une des revendications 1 à 18, en tant que substances actives pharmaceutiques pour le traitement et la prophylaxie de maladies infectieuses.

**23.** Procédé pour la préparation des composés selon l'une des revendications 1 à 18, caractérisé en ce que

(a) on fait réagir un composé de formule générale

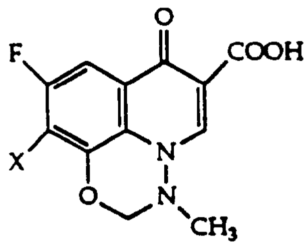

(II)

dans laquelle X représente un atome d'halogène; et des groupes amino, hydroxy et/ou carboxy présents peuvent être protégés,

avec une amine de formule générale

HR (III)

dans laquelle R a la signification donnée dans la revendication 1,

et, si nécessaire, on élimine un groupe protecteur présent, ou en ce que

(b) on fait réagir un composé de formule générale

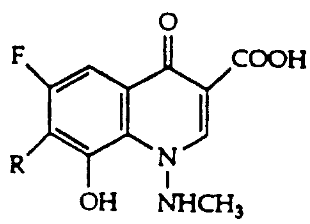

(IV)

dans laquelle R a la signification donnée dans la revendication 1, et des groupes amino, hydroxy et/ou carboxy présents peuvent être protégés,

avec du formaldéhyde ou paraformaldéhyde, et, si nécessaire, on élimine un groupe protecteur présent, ou en ce que

(c) pour la préparation d'un composé de formule I dans lequel R représente le groupe 4-acétyl-1-pipérazinyle, on fait réagir avec un agent donnant le groupe acétyle le composé de formule I dans lequel R représente le groupe 1-pipérazinyle non substitué, ou en ce que

(d) pour la préparation du composé de formule I dans lequel R représente le groupe 3-méthoxy-1-pipéridyle, on soumet à une méthylation le composé 3-hydroxy-1-pipéridyle de formule 1 correspondant, ou en ce que

(e) pour la préparation d'un composé de formule I comportant un groupe amino R libre, on élimine le groupe protecteur de fonction amino dans un composé de formule I correspondant, comportant un groupe amino protégé, ou en ce que

(f) pour la préparation du composé de formule I dans lequel R représente le groupe 3-chloro-1-pyrrolidinyle, on soumet à une halogénation le composé 3-hydroxy-1-pyrrolidinyle correspondant, à groupe carboxy protégé, et on élimine le groupe carboxy, ou en ce que

(g) pour la préparation du composé de formule I dans lequel R représente le groupe 4-(4-aminobenzyl)-1-pipérazinyle, on réduit le groupe nitro du composé 4-(4-nitrobenzyl)-1-pipérazinyle correspondant, ou en ce que

(h) pour la préparation de sels pharmaceutiquement acceptables, d'hydrates ou de produits de solvatation d'un composé de formule I ou d'hydrates et de produits de solvatation desdits sels, on convertit un composé de formule I en un sel, hydrate ou produit de solvatation ou en un hydrate ou produit de solvatation dudit sel.

**24.** Compositions pharmaceutiques, caractérisées par une teneur en un composé selon l'une des revendications 1 à 18.

**25.** Compositions pharmaceutiques pour le traitement et la prophylaxie de maladies infectieuses, caractérisées par une teneur en un composé selon l'une des revendications 1 à 18.

**26.** Utilisation de composés selon l'une des revendications 1 à 18, dans la fabrication de médicaments pour le traitement ou la prophylaxie de maladies infectieuses.

**Revendications pour l'Etat contractant suivant : GR**

**1.** Procédé pour la préparation de dérivés de pyrido[3,2,1-ij]-1,3,4-benzoxadiazine de formule générale

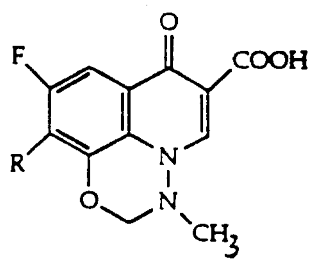

(I)

dans laquelle R représente
- un radical 1-pipérazinyle qui peut être substitué en position 4 par un groupe méthyle, acétyle ou 4-aminobenzyle;
- le radical morpholino;
- un radical 1-pyrrolidinyle substitué en position 3 par un atome de chlore ou par un groupe amino, aminométhyle, (méthylamino)méthyle, (éthylamino)méthyle ou méthoxy;
- un radical 1-imidazolyle qui peut être substitué en position 4 par le groupe méthyle; ou
- un radical 1-pipéridyle substitué en position 4 par un groupe hydroxy ou méthoxy,

ainsi que de leurs sels pharmaceutiquement acceptables et d'hydrates ou de produits de solvatation des composés de formule I ou de leurs sels, caractérisé en ce que

(a) on fait réagir un composé de formule générale

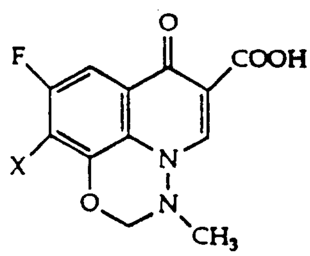

(II)

dans laquelle X représente un atome d'halogène; et des groupes amino, hydroxy et/ou carboxy présents peuvent être protégés,
avec une amine de formule générale

HR (III)

dans laquelle R a la signification donnée plus haut,
et, si nécessaire, on élimine un groupe protecteur présent, ou en ce que

(b) on fait réagir un composé de formule générale

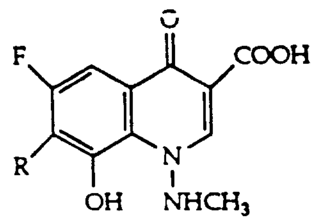

(IV)

dans laquelle R a la signification donnée plus haut, et des groupes amino, hydroxy et/ou carboxy présents peuvent être protégés,
avec du formaldéhyde ou paraformaldéhyde, et, si nécessaire, on élimine un groupe protecteur présent, ou en ce que
(c) pour la préparation d'un composé de formule I dans lequel R représente le groupe 4-acétyl-1-pipérazinyle, on fait réagir avec un agent donnant le groupe acétyle le composé de formule I dans lequel R représente le groupe 1-pipérazinyle non substitué, ou en ce que
(d) pour la préparation du composé de formule I dans lequel R représente le groupe 3-méthoxy-1-pipéridyle, on soumet à une méthylation le composé 3-hydroxy-1-pipéridyle de formule 1 correspondant, ou en ce que
(e) pour la préparation d'un composé de formule I comportant un groupe amino R libre, on élimine le groupe protecteur de fonction amino dans un composé de formule I correspondant, comportant un groupe amino protégé, ou en ce que
(f) pour la préparation du composé de formule I dans lequel R représente le groupe 3-chloro-1-pyrrolidinyle, on soumet à une halogénation le composé 3-hydroxy-1-pyrrolidinyle correspondant, à groupe carboxy protégé, et on élimine le groupe carboxy, ou en ce que
(g) pour la préparation du composé de formule I dans lequel R représente le groupe 4-(4-aminobenzyl)-1-pipérazinyle, on réduit le groupe nitro du composé 4-(4-nitrobenzyl)-1-pipérazinyle correspondant, ou en ce que
(h) pour la préparation de sels pharmaceutiquement acceptables, d'hydrates ou de produits de solvatation d'un composé de formule I ou d'hydrates et de produits de solvatation desdits sels, on convertit un composé de formule I en un sel, hydrate ou produit de solvatation ou en un hydrate ou produit de solvatation dudit sel.

**2.** Procédé pour la préparation de composés selon la revendication 1, dans lesquels R représente le groupe

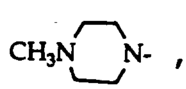

caractérisé en ce que l'on utilise des composes de départ substitués de façon correspondante.

**3.** Procédé pour la préparation de composés selon la revendication 1, dans lesquels R représente le groupe

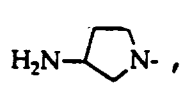

caractérisé en ce que l'on utilise des composés de départ substitués de façon correspondante.

**4.** Procédé pour la préparation d'un composé selon la revendication 1, à savoir de l'acide 9-fluoro-3-méthyl-10-(4-méthyl-1-pipérazinyl)-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ

substitués de façon correspondante.

5. Procédé pour la préparation d'un composé selon la revendication 1, à savoir de l'acide 9-fluoro-3-méthyl-7-oxo-10-(1-pipérazinyl)-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

6. Procédé pour la préparation d'un composé selon la revendication 1, à savoir de l'acide 10-[3-[-(éthylamino)méthyl]-1-pyrrolidinyl]-9-fluoro-3-méthyl-7-oxo-2,3-dihydro-7H-pyrido-[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

7. Procédé pour la préparation d'un composé selon la revendication 1, à savoir de l'acide 10-(3-amino-1-pyrrolidinyl)-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

8. Procédé pour la préparation d'un composé selon la revendication 1, à savoir de l'acide 9-fluoro-3-méthyl-10-[3-[(méthylamino)méthyl]-1-pyrrolidinyl]-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

9. Procédé pour la préparation d'un composé selon la revendication 1, à savoir de l'acide 10-[4-(4-aminobenzyl)-1-pipérazinyl]-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

10. Procédé pour la préparation d'un composé selon la revendication 1, à savoir de l'acide 9-fluoro-3-méthyl-10-morpholino-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

11. Procédé pour la préparation d'un composé selon la revendication 1, à savoir de l'acide 9-fluoro-10-(3-méthoxy-1-pyrrolidinyl)-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

12. Procédé pour la préparation d'un composé selon la revendication 1, à savoir de l'acide 10-[3-(aminométhyl)-1-pyrrolidinyl)-9-fluoro-3-méthyl-7-oxo-2,3-dihydro-7H-pyrido-[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

13. Procédé pour la préparation d'un composé selon la revendication 1, à savoir de l'acide 9-fluoro-10-(1-imidazolyl)-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

14. Procédé pour la préparation d'un composé selon la revendication 1, à savoir de l'acide 9-fluoro-3-méthyl-10-(4-méthyl-1-imidazolyl)-7-oxo-2,3-dihydro-7H-pyrido[3,2,1-ij]-benzoxadiazine-6-carboxylique

ou d'un sel pharmaceutiquement acceptable de celui-ci, ou d'un hydrate ou produit de solvatation de l'un de ces composés, caractérisé en ce que l'on utilise des composés de départ substitués de façon correspondante.

**15.** Procédé pour la préparation d'un composé selon la revendication 1, à savoir de l'acide 9-fluoro-10-(4-hydroxy- 1-pipéridyl)-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**16.** Procédé pour la préparation d'un composé selon la revendication 1, à savoir de l'acide 10-(3-chloro-1-pyrrolidinyl)-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**17.** Procédé pour la préparation d'un composé selon la revendication 1, à savoir de l'acide 10-(4-acétyl-1-pipérazinyl)-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**18.** Procédé pour la préparation d'un composé selon la revendication 1, à savoir de l'acide 9-fluoro-10-(4-méthoxy-1-pipéridyl)-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**19.** Procédé pour la préparation de compositions pharmaceutiquesn caractérisé en ce que l'on mélange un composé de formule I défini dans la revendication 1, ou un sel pharmaceutiquement acceptable de ce composé, ou un hydrate ou produit de solvatation d'un composé de formule I ou d'un sel de celui-ci, en tant que composant actif, avec des véhicules et/ou excipients solides ou liquides inertes, non toxiques, usuels en soi dans de telles compositions et appropriés à l'administration thérapeutique.

**20.** Utilisation de composés selon l'une des revendications 1 à 18, dans la fabrication de médicaments pour le traitement ou la prophylaxie de maladies infectieuses.

**21.** Composés de formule générale

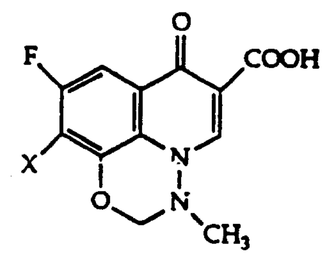

(II)

dans laquelle X représente un atome d'halogène.

**22.** Composés de formule générale

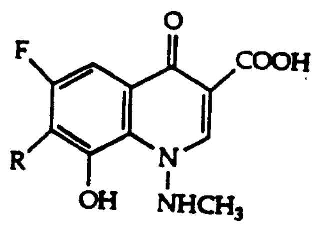

(IV)

dans laquelle R représente

- un radical 1-pipérazinyle qui peut être substitué en position 4 par un groupe méthyle, acétyle ou 4-aminobenzyle;
- le radical morpholino;
- un radical 1-pyrrolidinyle substitué en position 3 par un atome de chlore ou par un groupe amino, aminométhyle, (méthylamino)méthyle, (éthylamino)méthyle ou méthoxy;
- un radical 1-imidazolyle qui peut être substitué en position 4 par le groupe méthyle; ou
- un radical 1-pipéridyle substitué en position 4 par un groupe hydroxy ou méthoxy.

**Revendications pour les Etats contractants suivants : AT, ES**

**1.** Procédé pour la préparation de dérivés de pyrido[3,2,1-ij]-1,3,4-benzoxadiazine de formule générale

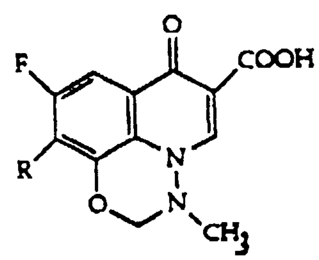

(I)

dans laquelle R représente

- un radical 1-pipérazinyle qui peut être substitué en position 4 par un groupe méthyle, acétyle ou 4-aminobenzyle;
- le radical morpholino;
- un radical 1-pyrrolidinyle substitué en position 3 par un atome de chlore ou par un groupe amino, aminométhyle, (méthylamino)méthyle, (éthylamino)méthyle ou méthoxy;
- un radical 1-imidazolyle qui peut être substitué en position 4 par le groupe méthyle; ou
- un radical 1-pipéridyle substitué en position 4 par un groupe hydroxy ou méthoxy,

ainsi que de leurs sels pharmaceutiquement acceptables et d'hydrates ou de produits de solvatation des composés de formule I ou de leurs sels, caractérisé en ce que

(a) on fait réagir un composé de formule générale

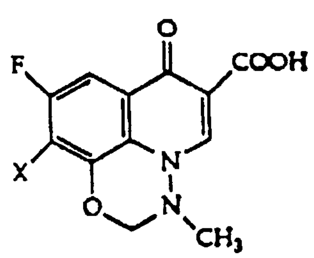

(II)

dans laquelle X représente un atome d'halogène; et des groupes amino, hydroxy et/ou carboxy

présents peuvent être protégés,
avec une amine de formule générale

HR (III)

dans laquelle R a la signification donnée plus haut,
et, si nécessaire, on élimine un groupe protecteur présent, ou en ce que
(b) on fait réagir un composé de formule générale

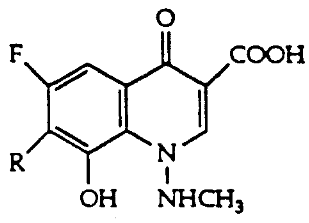

(IV)

dans laquelle R a la signification donnée plus haut, et des groupes amino, hydroxy et/ou carboxy présents peuvent être protégés,
avec du formaldéhyde ou paraformaldéhyde, et, si nécessaire, on élimine un groupe protecteur présent, ou en ce que
(c) pour la préparation d'un composé de formule I dans lequel R représente le groupe 4-acétyl-1-pipérazinyle, on fait réagir avec un agent donnant le groupe acétyle le composé de formule I dans lequel R représente le groupe 1-pipérazinyle non substitué, ou en ce que
(d) pour la préparation du composé de formule I dans lequel R représente le groupe 3-méthoxy-1-pipéridyle, on soumet à une méthylation le composé 3-hydroxy-1-pipéridyle de formule 1 correspondant, ou en ce que
(e) pour la préparation d'un composé de formule I comportant un groupe amino R libre, on élimine le groupe protecteur de fonction amino dans un composé de formule I correspondant, comportant un groupe amino protégé, ou en ce que
(f) pour la préparation du composé de formule I dans lequel R représente le groupe 3-chloro-1-pyrrolidinyle, on soumet à une halogénation le composé 3-hydroxy-1-pyrrolidinyle correspondant, à groupe carboxy protégé, et on élimine le groupe carboxy, ou en ce que
(g) pour la préparation du composé de formule I dans lequel R représente le groupe 4-(4-aminobenzyl)-1-pipérazinyle, on réduit le groupe nitro du composé 4-(4-nitrobenzyl)-1-pipérazinyle correspondant, ou en ce que
(h) pour la préparation de sels pharmaceutiquement acceptables, d'hydrates ou de produits de solvatation d'un composé de formule I ou d'hydrates et de produits de solvatation desdits sels, on convertit un composé de formule I en un sel, hydrate ou produit de solvatation ou en un hydrate ou produit de solvatation dudit sel.

**2.** Procédé pour la préparation de composés selon la revendication 1, dans lesquels R représente le groupe

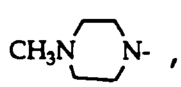

caractérisé en ce que l'on utilise des composés de départ substitués de façon correspondante.

**3.** Procédé pour la préparation de composés selon la revendication 1, dans lesquels R représente le groupe

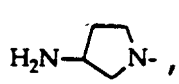

caractérisé en ce que l'on utilise des composés de départ substitués de façon correspondante.

**4.** Procédé pour la préparation d'un composé selon la revendication 1, à savoir de l'acide 9-fluoro-3-méthyl-10-(4-méthyl-1-pipérazinyl)-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**5.** Procédé pour la préparation d'un composé selon la revendication 1, à savoir de l'acide 9-fluoro-3-méthyl-7-oxo-10-(1-pipérazinyl)-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**6.** Procédé pour la préparation d'un composé selon la revendication 1, à savoir de l'acide 10-[3-[(éthylamino)-méthyl]-1-pyrrolidinyl]-9-fluoro-3-méthyl-7-oxo-2,3-dihydro-7H-pyrido-[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**7.** Procédé pour la préparation d'un composé selon la revendication 1, à savoir de l'acide 10-(3-amino-1-pyrrolidinyl)-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**8.** Procédé pour la préparation d'un composé selon la revendication 1, à savoir de l'acide 9-fluoro-3-méthyl-10-[3-[(méthylamino)méthyl]-1-pyrrolidinyl]-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**9.** Procédé pour la préparation d'un composé selon la revendication 1, à savoir de l'acide 10-[4-(4-aminobenzyl)-1-pipérazinyl]-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**10.** Procédé pour la préparation d'un composé selon la revendication 1, à savoir de l'acide 9-fluoro-3-méthyl-10-morpholino-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**11.** Procédé pour la préparation d'un composé selon la revendication 1, à savoir de l'acide 9-fluoro-10-(3-méthoxy-1-pyrrolidinyl)-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**12.** Procédé pour la préparation d'un composé selon la revendication 1, à savoir de l'acide 10-[3-(aminométhyl)-1-pyrrolidinyl)-9-fluoro-3-méthyl-7-oxo-2,3-dihydro-7H-pyrido-[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**13.** Procédé pour la préparation d'un composé selon la revendication 1, à savoir de l'acide 9-fluoro-10-(1-imidazolyl)-3-méthyl-7-oxo-2,3-dihydro-7H-pyrido[3,2,1-ij]-1,3,4-benzoxadiazine-6-carboxylique ou d'un

sel pharmaceutiquement acceptable de celui-ci, ou d'un hydrate ou produit de solvatation de l'un de ces composés, caractérisé en ce que l'on utilise des composés de départ substitués de façon correspondante.

**14.** Procédé pour la préparation d'un composé selon la revendication 1, à savoir de l'acide 9-fluoro-3-méthyl-10-(4-méthyl-1-imidazolyl)-7-oxo-2,3-dihydro-7H-pyrido[3,2,1-ij]-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**15.** Procédé pour la préparation d'un composé selon la revendication 1, à savoir de l'acide 9-fluoro-10-(4-hydroxy- 1-pipéridyl)-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**16.** Procédé pour la préparation d'un composé selon la revendication 1, à savoir de l'acide 10-(3-chloro-1-pyrrolidinyl)-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**17.** Procédé pour la préparation d'un composé selon la revendication 1, à savoir de l'acide 10-(4-acétyl-1-pipérazinyl)-9-fluoro-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**18.** Procédé pour la préparation d'un composé selon la revendication 1, à savoir de l'acide 9-fluoro-10-(4-méthoxy-1-pipéridyl)-3-méthyl-7-oxo-2,3-dihydro-7H-pyrido[3,2,1-ij]-1,3,4-benzoxadiazine-6-carboxylique ou d'un sel pharmaceutiquement acceptable de celui-ci, ou d'un hydrate ou produit de solvatation de l'un de ces composés, caractérisé en ce que l'on utilise des composés de départ substitués de façon correspondante.

**19.** Procédé pour la préparation de compositions pharmaceutiquesn caractérisé en ce que l'on mélange un composé de formule I défini dans la revendication 1, ou un sel pharmaceutiquement acceptable de ce composé, ou un hydrate ou produit de solvatation d'un composé de formule I ou d'un sel de celui-ci, en tant que composant actif, avec des véhicules et/ou excipients solides ou liquides inertes, non toxiques, usuels en soi dans de telles compositions et appropriés à l'administration thérapeutique.

**20.** Utilisation de composés selon l'une des revendications 1 à 18, dans la fabrication de médicaments pour le traitement ou la prophylaxie de maladies infectieuses.